# EUROPEAN PATENT APPLICATION

(11) **EP 3 266 869 A1**
(43) Date of publication of application: **10.01.2018**
(21) Application number: 16758764.1
(22) Date of filing: 18.02.2016
(51) Int. Cl.: C12N 15/09, C12M 1/00, C12Q 1/68, G01N 21/78

(54) **METHOD FOR ANALYZING TEMPLATE NUCLEIC ACID**

(30) Priority: 03.03.2015 JP 2015041813
(71) Applicant: Kabushiki Kaisha DNAFORM, Yokohama-shi Kanagawa 230-0046 (JP)
(72) Inventor: TSUJIMARU, Koichiro, Yokohama-shi Kanagawa 230-0046 (JP)
(74) Representative: J A Kemp
(86) International application number: PCT/JP2016/054718
(87) International publication number: WO 2016/140070

(57) **Abstract**

The present invention provides a method for accurately analyzing a template nucleic acid. The method is a method for analyzing a template nucleic acid including: an amplification step of amplifying a target sequence in the template nucleic acid and a complementary sequence to the target sequence; and a detection step of detecting association between a probe and an amplification product obtained in the amplification step or dissociation of an assembly between the probe and the amplification product with a temperature change. A primer set is used in the amplification step, and the primer set is a combination of a first primer for a polymerase that performs priming at the 3' end of a primer to synthesize the target sequence and a second primer for the polymerase to synthesize the complementary strand. Between the first primer and the second primer, a primer that initiates synthesis of a sequence to be hybridized with the probe is a turnback primer including a primer region and a probe region being directly or indirectly linked to the 5' end of the primer region, and the other primer is any primer having an ability of causing the polymerase to perform priming at the 3' end. In the amplification step, the turnback primer extends the target sequence or the complementary sequence from the primer region. In the detection step, the turnback primer also serves as the probe, and association between the probe region and the target sequence or the complementary sequence, occurred by turning the probe region of the turnback primer in a molecule of the amplification product back toward the extended target sequence or the extended complementary sequence, is detected

## Description

### Technical Field

The present invention relates to a method for analyzing a template nucleic acid.

### Background Art

In gene analysis, an analysis method using amplification of a template nucleic acid using a primer and hybridization between the obtained amplification product and a probe is widely used. In this analysis method, analysis of the presence or absence of a target sequence (qualitative analysis), analysis of the amount of the target sequence (quantitative analysis), and typing of a polymorphism site in the target sequence can be performed, for example. In the typing, the kind (e.g., whether a wild type or a mutant type) and the type of conjugation (whether homozygous or heterozygous) of the base of the polymorphism site can be determined, for example.

The qualitative analysis and the quantitative analysis can be performed as follows, for example. A target sequence in the template nucleic acid is amplified using a primer set. The obtained amplification product is then brought into contact with a probe to be hybridized with the target sequence, and association between them (formation of a double strand by hybridization) or dissociation of the assembly is detected. The presence or absence of the target sequence can be determined according to the presence or absence of the association or the dissociation, and the amount of the target sequence can be determined by the amount of the association or the dissociation.

As the probe to be hybridized with the target sequence in the typing, a probe that is fullmatch or mismatch to the polymorphism site according to the kind of the base of the polymorphism site is used. The probe having a fullmatch shows higher specificity, i.e., a higher Tm, than the probe having a mismatch. Thus, the probe and the target sequence are brought into contact with each other, the temperature is changed from a low temperature region in the association state to a high temperature region, dissociation of the assembly with a temperature increase is detected as a signal, and whether the dissociation occurred at a Tm for the probe having a mismatch or a Tm for the probe having a fullmatch is determined, whereby the polymorphism site in the target sequence can be typed. The determination is usually performed using a dissociation curve (primary differential curve) obtained by plotting primary differential values of variations in time and signal. That is, the presence or absence of a peak(s) showing dissociation at the Tm for the probe having a mismatch and the Tm for the probe having a fullmatch is determined. When a peak is found at the Tm for the probe having a mismatch, which is a lower temperature (T_{L}), the target sequence is determined as having a mismatch base of the polymorphism site. When a peak is found at the Tm for the probe having a fullmatch, which is a higher temperature (T_{H}), the target sequence is determined as having a fullmatch base of the polymorphism site. When one peak is found, the base of the polymorphism site is determined as a homozygous base, and when two peaks are found, the base of the polymorphism site is determined as a heterozygous base.

However, such analysis method using the amplification and the hybridization of the probe involves the following problems.

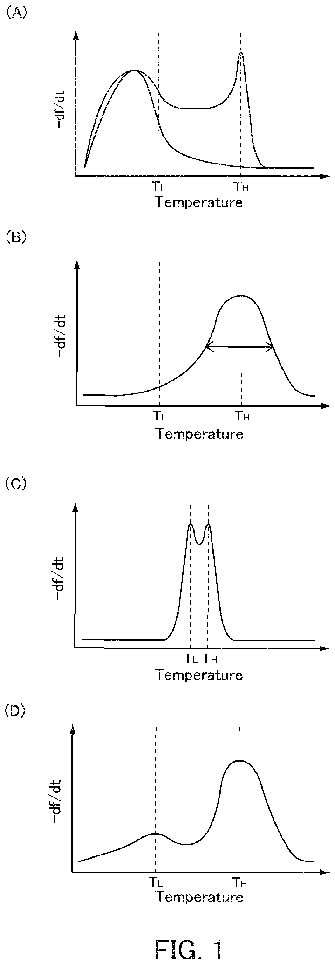

The first problem is an increase in background noise due to a nonspecific bond of the probe. That is, in the case where the probe hybridizes with a non-target sequence other than the target sequence, this association or dissociation of the assembly is also detected, and the background noise is thus increased. When a target sequence has a fullmatch, homogeneous polymorphism site in the typing, only one peak is found at a higher temperature (T_{H}) in the melting curve. However, as shown in (A) in FIG. 1, an increase in background noise is found in a low temperature region in some cases both in the control and the target sequence having a fullmatch homogeneous polymorphism site.

The second problem is a wide half width of a peak, i.e., an unsharp peak in the melting curve. As shown in (B) in FIG. 1, a peak obtained in the melting curve is unsharp, and it is thus difficult to determine whether the peak is specific to the predetermined temperature (T_{H}) in some cases.

The third problem is that a small distance between a peak of the target sequence having a fullmatch and a peak of a target sequence having a mismatch in the typing. As shown in (C) in FIG. 1, the distance between the peaks is small, although the respective peaks are found at a temperature (T_{L}) and a temperature (T_{H}), and it is thus difficult to determine which of the peaks is at which of the temperatures and to determine whether the number of peaks is one or two in some cases.

The fourth problem is an insufficient degree of separation in the typing of the heterozygous state. As shown in (D) in FIG. 1, a peak at a lower temperature (T_{L}) is small, although another peak is found at a higher temperature (T_{H}), and it is thus difficult to determine whether the target sequence is heterozygous or not in some cases.

### Summary of Invention

### Problem to be Solved by the Invention

Hence, the present invention is intended to provide, for example, a method for detecting a nucleic acid mutation in a nucleic acid mutation site with higher accuracy. Means for Solving Problem

In order to achieve the aforementioned object, the present invention provides a method for analyzing a template nucleic acid, including: an amplification step of amplifying a target sequence in the template nucleic acid and a sequence complementary to the target sequence; and a detection step of detecting association between a probe and an amplification product obtained in the amplification step or dissociation of an assembly between the probe and the amplification product, with a temperature change, a pH change, a concentration change of a denaturant, or a salt concentration change, wherein, the analysis method satisfies at least one of the following conditions (A) to (E):
(A) a primer set is used in the amplification step, the primer set is a combination of a first primer for a polymerase that performs priming at the 3' end of a primer to synthesize the target sequence and a second primer for the polymerase to synthesize the complementary strand, between the first primer and the second primer, a primer that initiates synthesis of a sequence to be hybridized with the probe is a turnback primer including a primer region and a probe region being directly or indirectly linked to the 5' end of the primer region, and the other primer is any primer having an ability of causing the polymerase to perform priming at the 3' end, in the amplification step, the turnback primer extends the target sequence or the complementary sequence from the primer region, and in the detection step, the turnback primer also serves as the probe, and association between the probe region and the target sequence or the complementary sequence, which occurred by turning the probe region of the turnback primer in a molecule of the amplification product back toward the extended target sequence or the extended complementary sequence is detected;
(B) the detection step includes a step of detecting association between the probe and the amplification product with a temperature change while changing a temperature from a high temperature point to a low temperature point;
(C) in the amplification step, a primer set is used, the primer set is a combination of a first primer that performs priming of a polymerase that synthesizes the target sequence and a second primer that performs priming of a polymerase that synthesizes the complementary strand, and between the first primer and the second primer, a primer that performs priming of a polymerase that synthesizes a sequence to be hybridized with the probe or the probe region in the condition (A) is used in an amount higher than the other primer;
(D) in the probe, the length of a region to be hybridized with the target sequence or the complementary sequence is 6 to 100 nucleotides; and
(E) the primer set is a combination of a first primer that performs priming of a polymerase that synthesizes the target sequence and a second primer that performs priming of a polymerase that synthesizes the complementary sequence, the first primer has the same sequence as an upstream region at any site of the template nucleic acid, the second primer has a complementary sequence to a downstream region at any site of the template nucleic acid, and the analysis method further satisfies the condition (e1) or (e2):
   (e1) the probe hybridizes with the target sequence, the 3' end of the upstream region in the template nucleic acid is positioned in the vicinity of the upstream side of the 5' end of a region to be hybridized with the probe in the template sequence, and the 5' end of the downstream region in the template nucleic acid is positioned in the vicinity of the downstream side of the 3' end of a region to be hybridized with the probe in the template sequence; and
   (e2) the probe hybridizes with the complementary sequence, the 3' end of the upstream region in the template nucleic acid is positioned in the vicinity of the upstream side of the 5' end of a region that overlaps in sequence with the probe in the template nucleic acid, and the 5' end of the downstream region in the template nucleic acid is positioned in the vicinity of the downstream side of the 3' end of a region that overlaps in sequence with the probe in the template nucleic acid.

The present invention further provides an oligonucleotide for performing the analysis method of the present invention.

The present invention further provides a device for performing the analysis method of the present invention.

### Effects of the Invention

According to the present invention, the template nucleic acid can be analyzed accurately.

### Brief Description of Drawings

[FIG. 1] FIG1 shows drawings illustrating example melting curves.
[FIG. 2] FIG. 2 shows drawings illustrating example analysis curves according to the analysis method of the present invention.
[FIG. 3] FIG. 3 is a drawing schematically illustrating design of a primer and a probe in the present invention.
[FIG. 4] FIG. 4 shows drawings schematically illustrating a turnback primer in the present invention.

### Description of Embodiments

For example, the analysis method of the present invention is a method for analyzing a nucleic acid mutation present in a target nucleic acid mutation site in the template nucleic acid. The target sequence has the nucleic acid mutation site in the template nucleic acid. The amplification step is a step of amplifying the target sequence and a complementary sequence to the target sequence. The detection step is a step of detecting association between the probe and the amplification product obtained in the amplification step or dissociation of an assembly between the probe and the amplification product, with a temperature change, a pH change, a concentration change of a denaturant, or a salt concentration change. The probe hybridizes with the target sequence or the complementary sequence, and a nucleic acid mutation at the nucleic acid mutation site can be detected on the basis of a difference in dissociation temperature or association temperature between the probe that is mismatch to the nucleic acid mutation site and the probe that is fullmatch to the same or between the probe that is mismatch to the nucleic acid mutation site and the probe that is different mismatch having a different Tm.

For example, the analysis method of the present invention satisfies the condition (B), and the high temperature point and the low temperature point satisfy the following condition (b1) or (b2):
(b1) the high temperature point is defined as any temperature point in a higher temperature region than the Tm in hybridization between "the probe" and "the target sequence having a nucleic acid mutation site to which the probe shows mismatch", and the low temperature point is defined as any temperature point in a lower temperature region than the Tm in hybridization between "the probe" and "the target sequence having a nucleic acid mutation site to which the probe shows fullmatch"; and
(b2) in the case where the probe shows mismatch to the nucleic acid mutation site and the probe shows different mismatch having a different Tm, the high temperature point is any temperature point in a higher temperature region than a relatively low Tm between the two Tms, and the lower temperature point is any temperature point in a lower temperature region than the relatively high Tm between the two Tms.

For example, the analysis method of the present invention satisfies the condition (E), the any site is the nucleic acid mutation site, the first primer has the same sequence as the upstream region of the nucleic acid mutation site in the template nucleic acid, and the second primer has a complementary sequence to the downstream region of the nucleic acid mutation site in the template nucleic acid.

In the analysis method of the present invention, a method of the amplification in the amplification step is, for example, PCR.

In the analysis method of the present invention, the probe is, for example, a fluorogenic probe.

For example, in the analysis method of the present invention, the probe includes at least two fluorescent atomic groups that exhibit an excitonic effect, and the two fluorescent atomic groups have a structure bound to the same base or the respective two bases adjacent to each other in the probe via a linker.

For example, in the analysis method of the present invention, the detection step is performed in the presence of an intercalator, and the intercalator is a fluorescent dye that develops color by intercalation of the intercalator to a double helix structure formed by hybridization of the probe.

For example, the analysis method of the present invention satisfies any one of the following combinations of the conditions (A) to (E):
the combination of the conditions (A) and (B);
the combination of the conditions (A), (B), and (C);
the combination of the conditions (A), (B), (C), and (D);
the combination of the conditions (A), (B), (C), (D), and (E);
the combination of the conditions (A) and (C);
the combination of the conditions (A), (C), and (D);
the combination of the conditions (A), (C), (D), and (E);
the combination of the conditions (A) and (D);
the combination of the conditions (A), (D), and (E);
the combination of the conditions (A) and (E);
the combination of the conditions (B) and (C);
the combination of the conditions (B), (C), and (D);
the combination of the conditions (B), (C), (D), and (E);
the combination of the conditions (B) and (D);
the combination of the conditions (B), (D), and (E); and
the combination of the conditions (B) and (E).

In the analysis method of the present invention, the detection step is, for example, performed in the presence of at least one additive selected from the group consisting of DMSO, betaine, urea, formamide, and formalin.

For example, the analysis method of the present invention satisfies the condition (A) and further includes: a step of performing the amplification step in the presence of a nickase or adding a nickase after the amplification step to design a nickase recognition sequence in "the turnback primer or a nucleic acid strand synthesized of the turnback primer" and nick a complementary strand to "the turnback primer or the nucleic acid strand synthesized of the turnback primer".

For example, the analysis method of the present invention satisfies the condition (A), and in the turnback primer, the probe region is indirectly linked to the 5' end of the primer region via an intervening sequence.

In the analysis method of the present invention, the nickase recognition sequence is present in, for example, the intervening sequence.

For example, the analysis method of the present invention satisfies the condition (A) and further satisfies the condition (a1), (a1), (a2), or (a2'): (a1) in the case where the first primer is the turnback primer and an intervening sequence is absent between the primer region (Ac') and the probe region (B'), (X - Y)/(X) is in the range from -1 to 1, assuming that the number of bases of the primer region (Ac') is X, and the number of bases of a region sandwiched between a region (A) to be hybridized with the primer region (Ac') and a region (B) to be hybridized with the probe (B') in the complementary sequence is Y; (a1') in the case where the first primer is the turnback primer, and an intervening sequence is present between the primer region (Ac') and the probe region (B'), [X - (Y - Y) ]/X is in the range from -1 to 1, assuming that the number of bases of the primer region (Ac') is X, the number of bases of a region sandwiched between a region (A) to be hybridized with the primer region (Ac') and a region (B) to be hybridized with the probe (B') is Y, and the number of bases of the intervening sequence is Y; (a2) in the case where the second primer is the turnback primer, and an intervening sequence is absent between the primer region (cAc') and the probe region (cB'), (X - Y) /(X) is in the range from -1 to 1, assuming that the number of bases of the primer region (cAc') is X, and the number of bases of a region sandwiched between a region (cA) to be hybridized with the primer region (cAc') and a region (cB) to be hybridized with the probe (cB') in the target sequence is Y; and (a2') in the case where the second primer is the turnback primer, and an intervening sequence is present between the primer region (cAc') and the probe region (cB'), [X - (Y - Y')]/X is in the range from -1 to 1, assuming that the number of bases of the primer region (cAc') is X, the number of bases of a region sandwiched between a region (cA) to be hybridized with the primer region (cAc') and a region (cB) to be hybridized with the probe (cB') in the target sequence is Y, and the number of bases in the intervening sequence is Y'.

For example, the analysis method of the present invention satisfies the condition (C), and X is in the range of 1 < X ≤ 10⁶, assuming that the concentration of a primer (M) is higher than that of the other primer (S), and a molar ratio (M : S) is X : 1.

For example, in the analysis method of the present invention, the probe is an FRET probe including at least two fluorescent atomic group pairs that exhibit an excitonic effect or an FRET probe including at least one fluorescent atomic group pair that exhibits an excitonic effect and a dye that exhibits no excitonic effect, and the two fluorescent atomic groups of each of the fluorescent dy moiety pairs are bound to the same base or the respective bases that are adjacent to each other in the probe via linkers and are arranged to exhibit an FRET effect in the probe.

In the analysis method of the present invention, the probe includes, for example, at least one of a non-nucleotide residue, a modified nucleotide residue, and a non-natural main backbone.

In the analysis method of the present invention, the non-natural main backbone is, for example, at least one of LNA, PNA, and a nucleic acid having a modified phosphodiester bond.

For example, the analysis method of the present invention satisfies at least one of the conditions (A) and (C) to (E), and the detection step is a step of detecting dissociation of an assembly between the probe and the amplification product with a temperature change while changing a temperature from a low temperature point to a high temperature point. In this case, the high temperature point and the low temperature point satisfy the following condition (b1) or (b2):
(b1) the high temperature point is defined as any temperature point in a higher temperature region than the Tm in hybridization between "the probe" and "the target sequence having a nucleic acid mutation site to which the probe shows mismatch", and the low temperature point is defined as any temperature point in a lower temperature region than the Tm in hybridization between "the probe" and "the target sequence having a nucleic acid mutation site to which the probe shows fullmatch"; and
(b2) in the case where the probe shows mismatch to the nucleic acid mutation site and the probe shows different mismatch having a different Tm, the high temperature point is any temperature point in a higher temperature region than a relatively low Tm between the two Tms, and the lower temperature point is any temperature point in a lower temperature region than the relatively high Tm between the two Tms.

The present invention is described below with reference to specific examples. The present invention, however, is not limited by the following description.

The present invention can be applied to analysis of a template nucleic acid using detection of association between an amplification product obtained using a primer and a probe or dissociation of its assembly, and the range of the application of the present invention is not limited to particular ranges.

The first example of the analysis of a template nucleic acid in the present invention can be analysis of the presence or absence (qualitative analysis) or the amount (quantitative analysis) of the template nucleic acid. In this case, the target sequence and the complementary sequence in the template nucleic acid are amplified, and the association of the obtained amplification product and the probe or the dissociation of the assembly is detected. Thus, the presence or absence or the amount of the template nucleic acid can be analyzed.

The second example can be, for example, analysis of a nucleic acid mutation of the nucleic acid mutation site in the target nucleic acid, i.e., typing. The nucleic acid mutation site is, for example, a polymorphism site such as a single nucleotide polymorphism. Specific examples of the analysis of the nucleic acid mutation include distinguishing between the wild type and the mutant type in the nucleic acid mutation site, distinguishing a different mutant type in the nucleic acid mutation, distinguishing between homozygote and a heterozygote. In this case, the target sequence in the template nucleic acid and the complementary sequence thereof are amplified, and the association of the obtained amplification product and the probe or the dissociation of the assembly is detected. The nucleic acid mutation site can be typed according to the conditions (e.g., temperature, pH, concentration of denaturant, and salt concentration) under which the association or the dissociation occurs.

The target sequence is a sequence of a region including any site in the template sequence in the present invention, and the complementary sequence is a sequence complementary to the target sequence. The target sequence and the complementary sequence in the present invention are amplified in the amplification step. The any site is not limited to particular sites.

In the case where the present invention is applied to typing, the any site is, for example, a nucleic acid mutation site. The nucleic acid mutation site is, for example, a site including a nucleic acid mutation to be detected. In this case, the target sequence is a sequence of a region including the nucleic acid mutation in the template nucleic acid, and the complementary sequence is a sequence complementary to the target sequence.

In the present invention, the amplification step can be performed using a nucleic acid sample. The nucleic acid sample may be a single-stranded nucleic acid or a double-stranded nucleic acid, for example. In the former case, for example, the single-stranded nucleic acid is the template nucleic acid. In the latter case, for example, either one of single-stranded nucleic acids to be paired in the double-stranded nucleic acid may be set to a template nucleic acid including the target sequence. Specifically, for example, in the single-stranded nucleic acids to be paired, the sense strand may be set to a template nucleic acid including the target sequence, or an antisense strand may be set to a template nucleic acid having the target sequence.

In the present invention, an amplification method in the amplification step is not limited to particular methods. The amplification method may be, for example, a non-isothermal amplification method or an isothermal amplification method, unless otherwise shown. The non-isothermal amplification method can be, for example, PCR. Examples of the isothermal amplification method include SmartAmp (NATURE METHODS (2007) VOL. 4, NO. 3, p. 257, Japanese Patent No. 3897805), Strand Displacement Amplification ((SDA), JP H07-114718 A), an improved SDA (U.S. Pat. No. 5824517, WO 99/09211, WO 95/25180), Nucleic Acid Sequence Based Amplification ((NASBA), Japanese Patent No. 2650159), a Loop-Mediated Isothermal Amplification ((LAMP), Japanese Patent No. 3313358, Nucleic Acids Research, 2000, Vol. 28, No. 12, e63), Isothermal and Chimeric primer-initiated Amplification of Nucleic acids ((ICAN), WO 02/16639), a self-sustained sequence replication ((3SR), a transcription-mediated amplification (TMA) method, an Invader method, and a rolling cycle amplification (RCA) method.

The amplification step is preferably performed in a reaction solution. The reaction solution contains, for example, a region to be required, as appropriate, according to the kind of the amplification method. Examples of the reaction solution include, in addition to the nucleic acid sample (template nucleic acid), a primer set, an enzyme such as polymerase, a monomer nucleic acid (dNTP) for amplification, and a buffer solution.

The amplification method in the amplification step is preferably PCR. The steps and the conditions of the PCR are not limited to particular steps and conditions, and conventional steps and conditions can be employed. The PCR includes, for example, a step of dissociating a double-stranded nucleic acid, a step of annealing a primer to a single-stranded nucleic acid, and a step of extending the primer, and the conditions of the steps can be set, as appropriate.

The amplification step is, for example, performed using a primer set. The primer set is, for example, a combination of a first primer for a polymerase that performs priming from the 3' end of the primer to synthesize the target sequence and a second primer for the polymerase to synthesize the complementary sequence. The primer set can be, for example, set according to the sequence of the template nucleic acid.

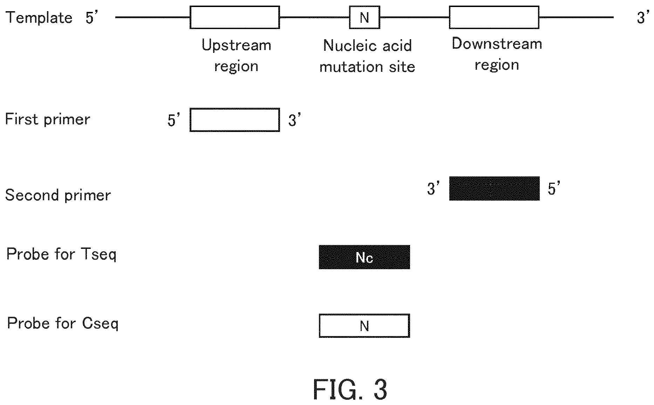

The primer set can be set with reference to FIG. 3, for example. FIG. 3 is a schematic view illustrating a relationship between the template nucleic acid and the primer set. In FIG. 3, each hollow region means the same sequence as the corresponding region of the template nucleic acid, and each black region means a sequence complementary to the corresponding region of the template nucleic acid (the same applies hereinafter). As shown in FIG. 3, the first primer has the same sequence as an upstream region of any site (e.g., the nucleic acid mutation site) of the template nucleic acid, for example. The sequence of the first primer may be, for example, only the same sequence or may include the same sequence and the other sequence. In the latter case, the first primer includes the same sequence preferably in the 3' end region. The second primer has a sequence complementary to a downstream region of any site (e.g., the nucleic acid mutation site) of the template nucleic acid. The sequence of the second primer may be, for example, only the complementary sequence or may include the complementary sequence and the other sequence. In the latter case, the second primer includes the complementary sequence preferably in the 3' end region.

The probe to be used in the detection step of the present invention may be a probe to be hybridized with the target sequence including any site or a probe to be hybridized with the complementary sequence. The probe can be set, as appropriate, according to the sequence of the template nucleic acid, for example. The former probe includes a sequence to be hybridized with the target sequence, for example. The sequence of the probe may be only the sequence to be hybridized with the target sequence or may include the sequence to be hybridized with the target sequence and the other sequence. The latter probe includes a sequence to be hybridized with the complementary sequence, for example. The sequence of the probe may be only the sequence to be hybridized with the complementary sequence or may include the sequence to be hybridized with the complementary sequence and the other sequence.

The probe can be set with reference to FIG. 3, for example. FIG. 3 is a schematic view illustrating a relationship between the template nucleic acid and the probe. As shown in FIG. 3, in the case where the probe is a probe (probe for Tseq) to be hybridized with the target sequence, the probe can be designed to include a sequence complementary to a region including any site N (e.g., the nucleic acid mutation site) in the template nucleic acid, for example. A portion corresponding to the any site N of the template nucleic acid in the probe for Tseq has Nc complementary to the any site N. In the case where the probe is a probe (probe for Cseq) to be hybridized with the complementary sequence, the probe can be designed to include the same sequence as a region including any site (for example, the nucleic acid mutation site) in the template nucleic acid.

In the case where the present invention is applied to typing, a base of the nucleic acid mutation site in the template nucleic acid may be set to, for example, a base of wild-type or a mutant-type and may be a base of any of the mutation types for a plurality of mutation types.

In the case where the present invention is applied to typing, the probe can be, for example, referred to as a probe for typing. The probe can be, for example, in the following first form or the following second form. The probe in the first form is a probe in which a dissociation temperature or an association temperature differs according to whether the probe is mismatch or fullmatch to the nucleic acid mutation site, and a nucleic acid mutation of the nucleic acid mutation site can be detected using the dissociation temperature or the association temperature. The probe in the first form allows whether the probe is mismatch or fullmatch to the nucleic acid mutation site to be determined using the dissociation temperature or the association temperature. Accordingly, whether the base at the nucleic acid mutation site is a target mutation (e.g., a mutant type or a wild type) can be detected. For example, the probe mt having a mutant-type nucleic acid mutation site shows a strong association force to the mutant-type target sequence mt having a fullmatch nucleic acid mutation site compared with the wild-type target sequence wt having a mismatch nucleic acid mutation site. The association temperature between the probe mt and the mutant-type target sequence mt is thus higher than that between the probe mt and the wild-type target sequence wt.

The probe in the second form is a probe in which a dissociation temperature or an association temperature differs according to whether the probe has a mismatch to the nucleic acid mutation site or a mismatch to the nucleic acid mutation site, having a difference in melting temperature (Tm) from the former mismatch, and a nucleic acid mutation of the nucleic acid mutation site can be detected using the dissociation temperature or the association temperature. The probe in the second form allows the type of the mismatch to the nucleic acid mutation site in the probe to be determined using the dissociation temperature or the association temperature. Accordingly, whether the base of the nucleic acid mutation site is a target mutation (e.g., the mutant-type or wild-type) can be detected.

In the detection step, association between the probe and the amplification product or dissociation of its assembly is detected. The probe preferably is a probe in which the behavior of a signal differs between the state of assembly and the state of dissociation of the assembly, for example. The state of the assembly is, for example, a state of forming a double strand (assembly) by hybridization between the probe and the amplification product, and the state of dissociation of the assembly is, for example, a state of single strands by releasing the probe from the double strand.

The difference in behavior of the signal may be, for example, a difference in that a signal is emitted in the association state, and a signal disappears or declines in the dissociation state or a difference in that a signal is emitted in the dissociation state, and a signal disappears or declined in the association state. In the present specification, the former difference, i.e., the difference in that a signal is emitted in the association state, and a signal disappears or declines in the dissociation state is used as an example. The difference, however, is by no means limited thereto, and the latter difference can be used.

The kind of the signal is not limited to particular kinds and is only required to be detected, and examples thereof include a fluorescence signal, a light emission signal, and a coloring signal. In the detection step, the association or the dissociation can be detected by measuring signal intensity, for example. In the present specification, the fluorescence signal is hereinafter used as an example of the signal, unless otherwise shown. The signal, however, is by no means limited thereto, and the light emission signal or the coloring signal can be sued.

The signal may be, for example, a signal emitted from the probe itself or a signal emitted from a reagent other than the probe. The probe that emits a signal is not limited to particular probes, and examples thereof include a fluorogenic probe and an exciton probe (Eprobe). The reagent that emits a signal can be, for example, an intercalator.

The fluorogenic probe is, for example, a probe that emits a fluorescence signal by formation of a double strand and is specifically, for example, a labeled probe to which a labeling substance that emits a signal is bound. The labeled probe can be, for example, a TaqMan (registered trademark) probe. In addition to the fluorogenic probe, a labeled probe such as a luminescence probe that emits a light emission signal by formation of a double strand or a coloring probe that emits a coloring signal by the same can be used as the probe in the present invention.

The Eprobe includes at least two fluorescent atomic groups that exhibit an excitonic effect, and the two fluorescent atomic groups are bound to the same base or the respective bases that are adjacent to each other in the probe via linkers, for example.

The E-probe can be described with reference to Japanese Patent No. 4370385 and WO 2014/013954, for example.

The Eprobe is a probe into which two fluorescent atomic groups (e.g. thiazole orange and its similar substance) have been introduced. The Eprobe has a property of hardly emitting fluorescence because of the excitonic effect obtained when two fluorescent atomic groups form exciplex in the case of single strand but strongly emitting fluorescence by dissociating the two fluorescent atomic groups and thus causing no excitonic effect at the time when the E-probe hybridizes with the target. Note here that while the "Eprobe" is a trade name of a product of Kabushiki Kaisha DNAFORM ("Eprobe" is a registered trademark), the "Eprobe" in the present invention may be identical to or different from the product given the trade name of the "Eprobe".

The 3' end of the Eprobe may be modified, for example, not to extend, and specifically, for example, the 3' end of the Eprobe may be chemically modified with a linker OH group.

In the Eprobe, fluorescent atomic groups that exhibit an excitonic effect are each:
(i) the one that emits fluorescence, with two planar chemical structures contained in one molecule, which exist not in the same plane but with a certain angle formed therebetween, being located so as to be arranged in the same plane when the molecule undergoes intercalation into or groove binding to a nucleic acid,
(ii) the one formed of at least two dye molecule groups that do not exhibit fluorescence emission due to the excitonic effect obtained when at least two dye molecules aggregate in parallel to each other but exhibit fluorescence emission with the aggregation state being resolved when the molecules undergo intercalation into or groove binding to a target molecule, e.g. a nucleic acid, or
(iii) the one characterized in having a chemical structure of at least two dye molecules contained in one molecule, with the at least two dye molecules not exhibiting fluorescence emission due to the excitonic effect obtained when they aggregate in parallel to each other but exhibiting fluorescence emission with the aggregation state being resolved when the molecules undergo intercalation into or groove binding to a target molecule, e.g. a nucleic acid.
In the case of (ii) or (iii), preferably, the dye molecule is the molecule described in (i).

In the nucleic acid probe of the present invention, the structure of the nucleic acid molecule may be, for example, a labeled nucleic acid containing at least one of the structures represented by the following formulae (16), (16b), (17), (17b), (18), and (18b). In the present invention, the labeled nucleic acid also encompasses tautomers and stereoisomers of these structures, as well as salts of these structures, tautomers, and stereoisomers. Hereinafter, the structures represented by the following respective formulae and having atomic groups Z¹¹ and Z¹² that exhibit fluorescence each may be referred to as a "labeled structure". The labeled nucleic acid containing the labeled structure may be referred to as a "labeled probe".

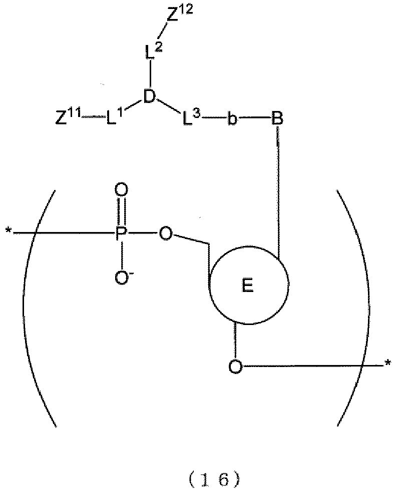

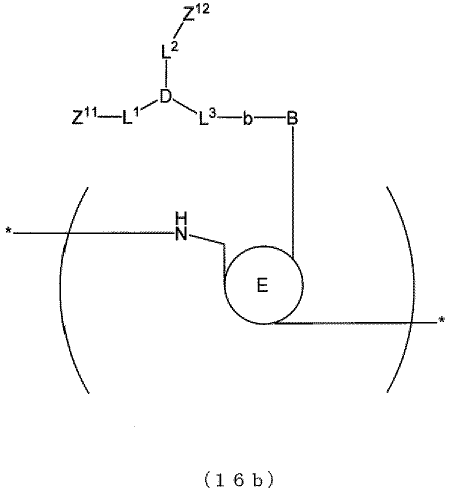

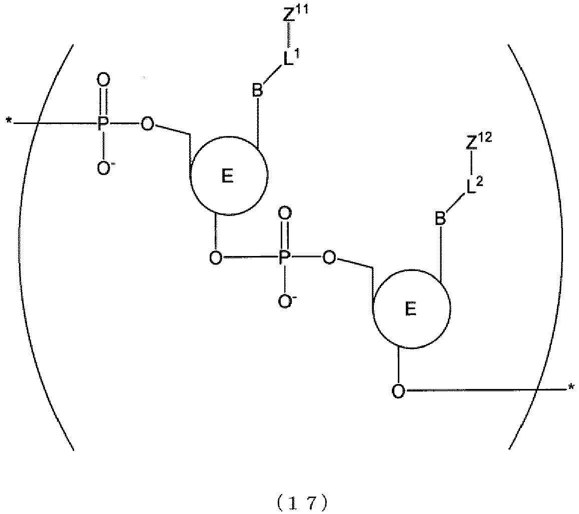

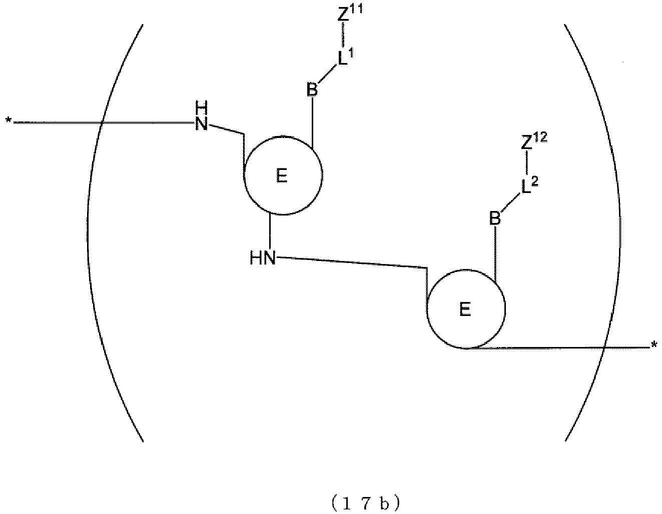

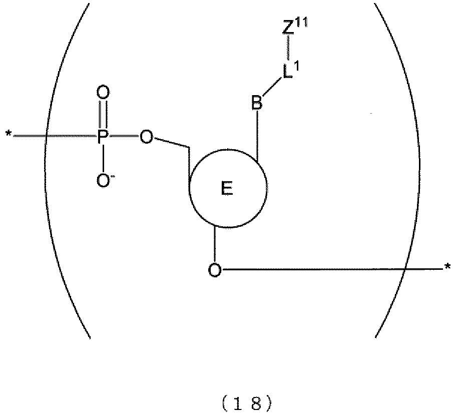

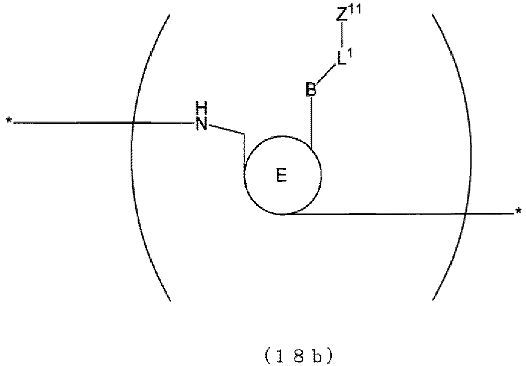

In the formulae (16), (16b), (17), (17b), (18), and (18b), B is an atomic group having a natural nucleobase (adenine, guanine, cytosine, thymine, or uracil) backbone or an artificial nucleobase backbone,
E is:
(i) an atomic group having a deoxyribose backbone, a ribose backbone, or a structure derived from either one of them, or
(ii) an atomic group having a peptide structure or a peptoid structure,
Z¹¹ and Z¹² are each a fluorescent atomic group, and may be identical to or different from each other,
L¹, L², and L³ are each a linker (a linking atom or a linking atomic group), the main chain length (the number of main chain atoms) thereof is any length, L¹, L², and L³ each may or may not contain each of C, N, O, S, P, and Si in the main chain, L¹, L², and L³ each may or may not contain each of a single bond, a double bond, a triple bond, an amide bond, an ester bond, a disulfide bond, an imino group, an ether bond, a thioether bond, and a thioester bond in the main chain, and L¹, L², and L³ may be identical to or different from each other,
D is CR, N, P, P=O, B, or SiR where R is a hydrogen atom, an alkyl group, or any substituent, and
b is a single bond, a double bond, or a triple bond,
or alternatively,
in the formulae (16) and (16b), L¹ and L² are each a linker, L³, D, and b may not be present, and L¹ and L² may be bonded directly to B, provided that:
in the formulae (16), (17), and (18), E is an atomic group described in the condition (i), and at least one O atom in a phosphoric acid linkage may be substituted with an S atom;
in the formulae (16b), (17b), and (18b), E is an atomic group described in the condition (ii); and
in the formulae (17) and (17b), the respective Bs may be identical to or different from each other, and the respective Es may be identical to or different from each other.

In the formulae (16), (17), (16b), (17b), (18), and (18b), the main chain length (the number of main chain atoms) of each of L¹, L², and L³ preferably is an integer of 2 or more. The upper limit thereof is not particularly limited, and is, for example, 100 or less, more preferably 30 or less, and particularly preferably 10 or less.

Z¹¹ and Z¹² are fluorescent atomic groups that exhibit an excitonic effect. With this configuration, a greater environmental change in surroundings of fluorescent dyes is obtained when Z¹¹ and Z¹² bind to a target sequence, i.e., for example, a greater increase in fluorescence is obtained when a double helix structure is formed. This allows the double helix structure to be detected still more effectively.

Z¹¹ and Z¹² are only required to be fluorescent atomic groups that exhibit an excitonic effect and are not particularly limited. More preferably, Z¹¹ and Z¹² are, for example, each independently a group derived from any one of thiazole orange, oxazole yellow, cyanine, hemicyanine, other cyanine dyes, methyl red, azo dyes, and derivatives thereof. Furthermore, a group derived from any of other known dyes also can be used as appropriate. Many fluorescent dyes that change the fluorescence intensity by binding to nucleic acids such as DNA have been reported. In a typical example, it has been known that ethidium bromide exhibits strong fluorescence by intercalating into a double helix structure of DNA, and it is used frequently for DNA detection. Furthermore, fluorescent dyes whose fluorescence intensity can be controlled according to the microscopic polarity, such as pyrenecarboxyamide and prodan, also are known. The thiazole orange is a fluorescent dye with a benzothiazole ring and quinoline ring linked to each other with a methine group. It usually exhibits weak fluorescence but gives strong fluorescence emission by intercalating into DNA having a double helix structure. Other examples include dyes such as fluorescein and Cy3.

More preferably, Z¹¹ and Z¹² are each independently an atomic group represented by any one of the following formulae (7) to (9).

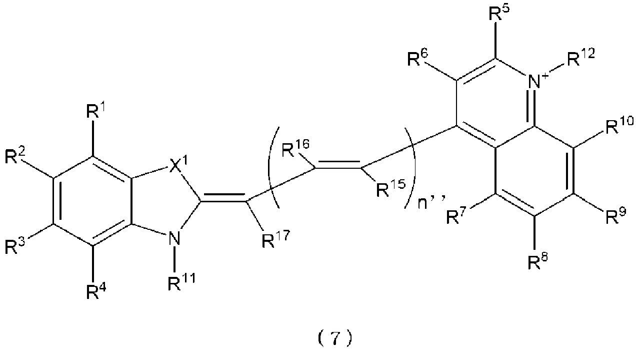

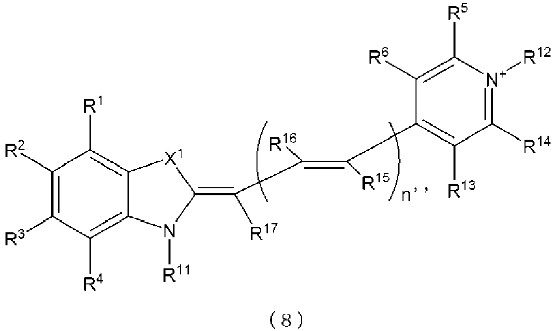

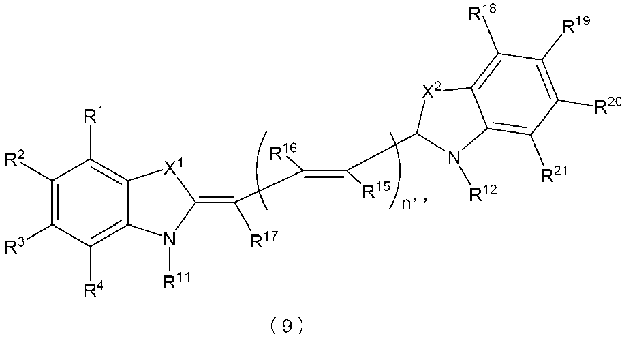

In the formulae (7) to (9),
X¹ and X² are each S, Se, or O,
n" is 0 or a positive integer,
R¹ to R¹⁰ and R¹³ to R²¹ are each independently a hydrogen atom, a halogen atom, a lower alkyl group, a lower alkoxyl group, a nitro group, or an amino group,
one of R¹¹ and R¹² is a linking group that is bound to L¹ or L² in the formulae (16), (17), (16b), (17b), (18), and (18b), and the other is a hydrogen atom or a lower alkyl group, when a plurality of R¹⁵s are present in the formula (7), (8), or (9), they may be identical to or different from each other,
when a plurality of R¹⁶s are present in the formula (7), (8), or (9), they may be identical to or different from each other, and
X¹, X², and R¹ to R²¹ in Z¹¹ and X¹, X², and R¹ to R²¹ in Z¹² may be identical to or different from each other, respectively.

In the formulae (7) to (9), it is more preferable that, in R¹ to R²¹, the lower alkyl group is a linear or branched alkyl group with a carbon number of 1 to 6, and the lower alkoxyl group is a linear or branched alkoxyl group with a carbon number of 1 to 6.

More preferably, in R¹¹ and R¹² in each of the formulae (7) to (9), the linking group is a polymethylene carbonyl group with a carbon number of at least 2 and is bound, in the carbonyl group moiety, to L¹ or L² in any of the formulae (16), (17), (16b), (17b), (18) and (18b). The upper limit of the carbon number of the polymethylene carbonyl group is not limited to particular values, and is, for example, 100 or less, preferably 50 or less more preferably 30 or less, and particularly preferably 10 or less.

When Z¹¹ and Z¹² are each represented by any of the formulae (7) to (9), they are, for example, more preferably each independently a group represented by the formula (19) or (20).

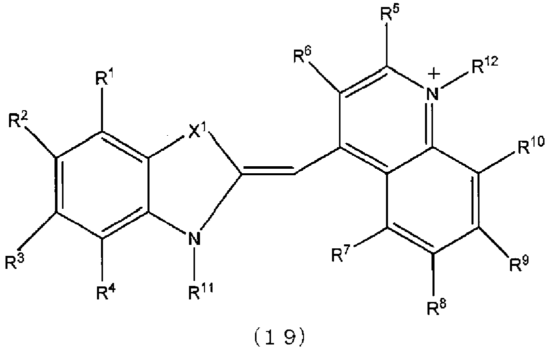

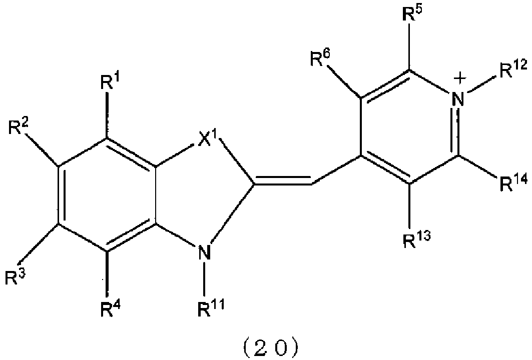

In the formulae (19) and (20), X¹ represents -S- or -O-. R¹ to R¹⁰ and R¹³ and R¹⁴ each independently represents a hydrogen atom, a halogen atom, a lower alkyl group, a lower alkoxyl group, a nitro group, or an amino group. One of R¹¹ and R¹² is a linking group that is bound to L¹ or L² in each of the formulae (16), (17), (16b), (17b), (18), and (18b), and the other is a hydrogen atom or a lower alkyl group.

Particularly preferably, Z¹¹ and Z¹² are each independently an atomic group represented by any one of the following chemical formulae.

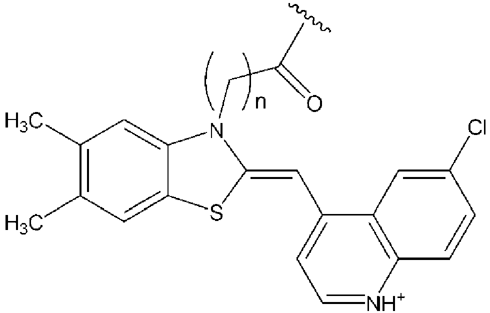

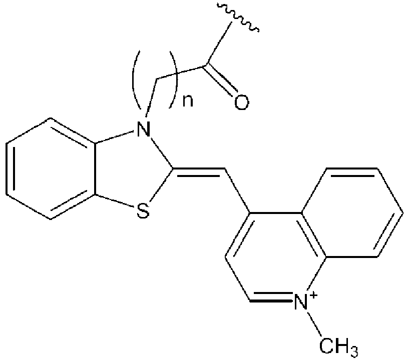

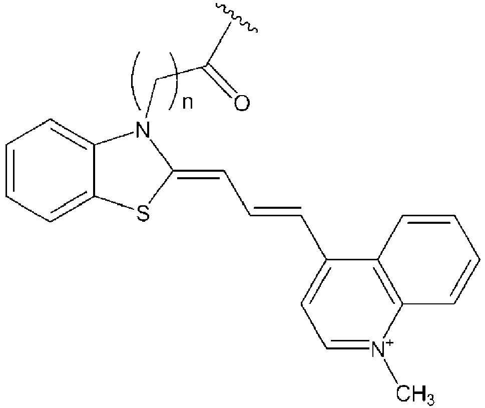

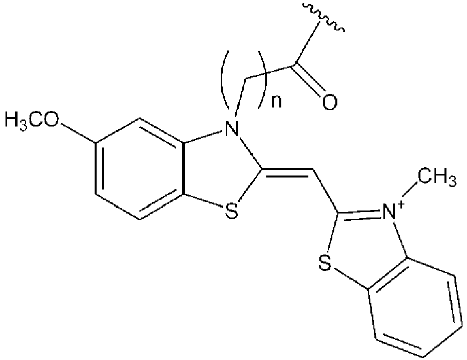

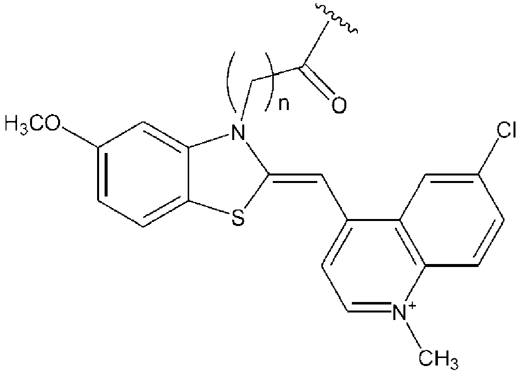

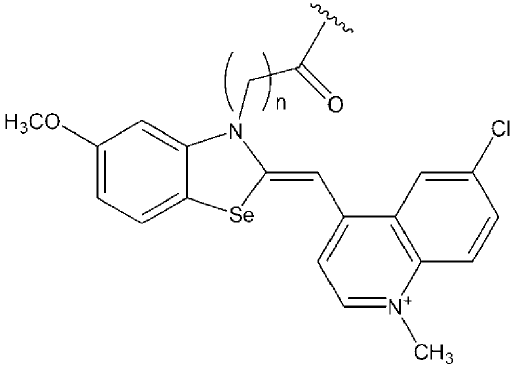

In each of the above chemical formulae, it is particularly preferable that n is a positive integer and in the range from 2 to 6.

In the formulae (16), (17), (16b), (17b), (18), and (18b), B may have a natural nucleobase backbone, and also, as described above, may have an artificial nucleobase backbone. For example, B preferably is a structure represented by Py (pyrimidine ring), Py der., Pu (purine ring), or Pu der. The Py is an atomic group having a covalent bond to E in the 1-position and a covalent bond to a linker moiety in the 5-position in a six-membered ring represented by the following formula (11). The Py der. is an atomic group in which at least one of all the atoms of the six-membered ring of the Py has been substituted with an N, C, S, or O atom, and the N, C, S, or O atom optionally may have an electric charge, a hydrogen atom, or a substituent. The Pu is an atomic group having a covalent bond to E in the 9-position and a covalent bond to a linker moiety in the 8-position in a condensed ring represented by the following formula (12). The Pu der. is an atomic group in which at least one of all the atoms of a five-membered ring of the Pu has been substituted with an N, C, S, or O atom, and the N, C, S, or O atom optionally may have an electric charge, a hydrogen atom, or a substituent.

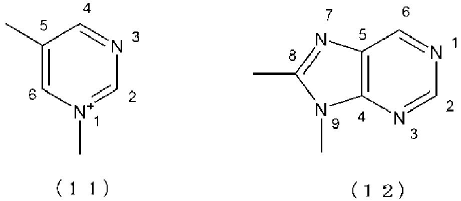

The nucleic acid molecule in the Eprobe may include, for example, at least one of the nucleotide structures represented by the following chemical formulae 106, 110, 113, 117, 120, 122, 123, 124, and 114-2, geometric isomers and stereoisomers thereof, and salts thereof.

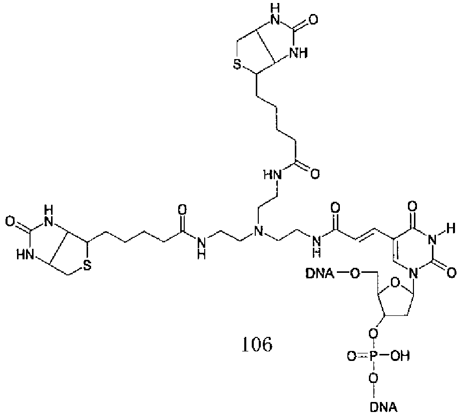

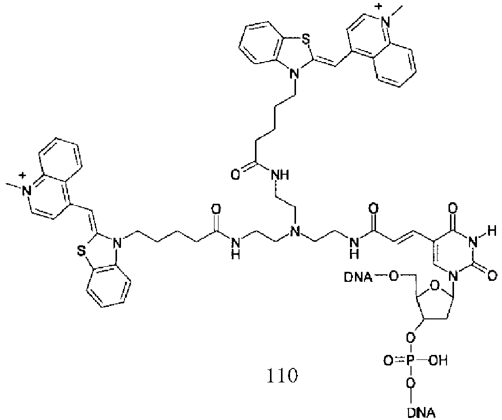

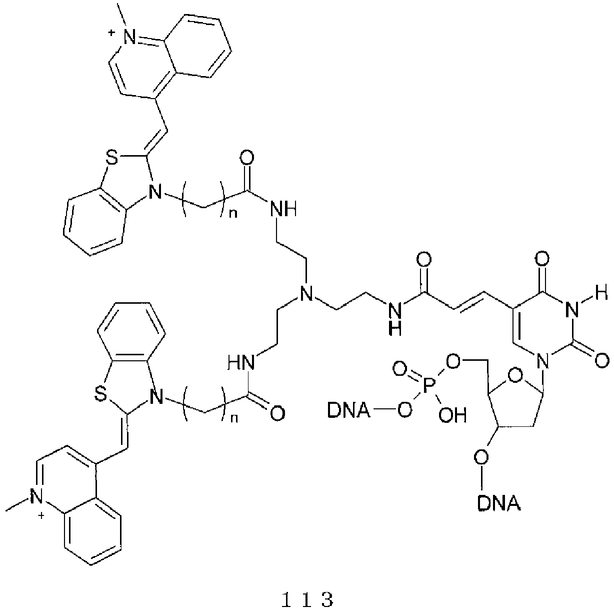

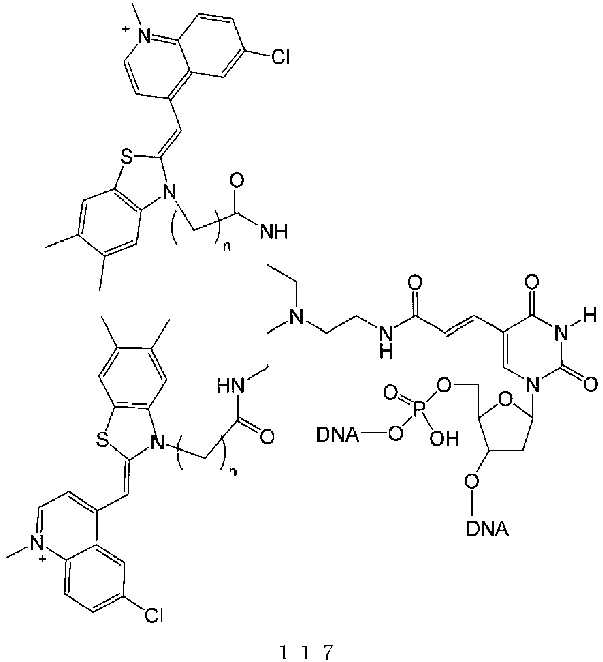

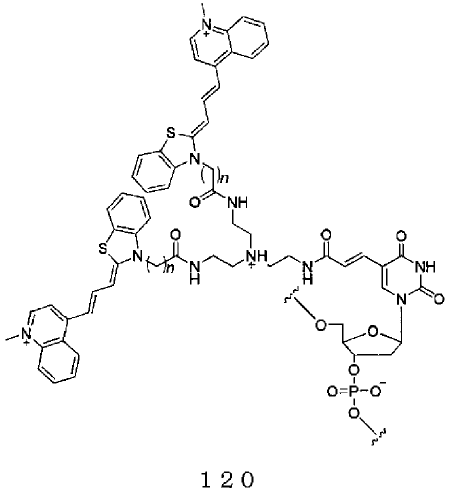

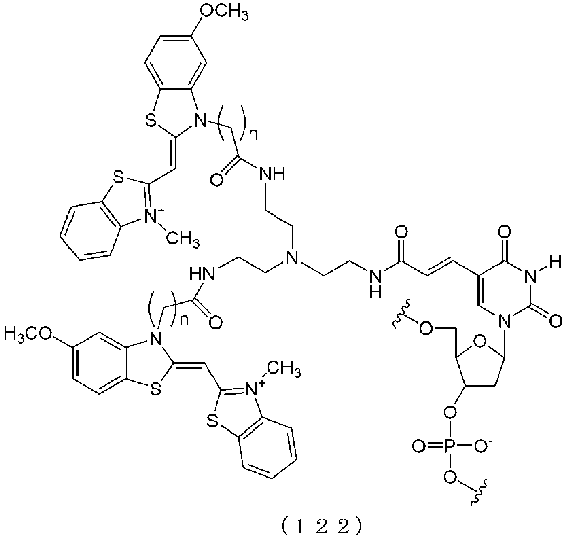

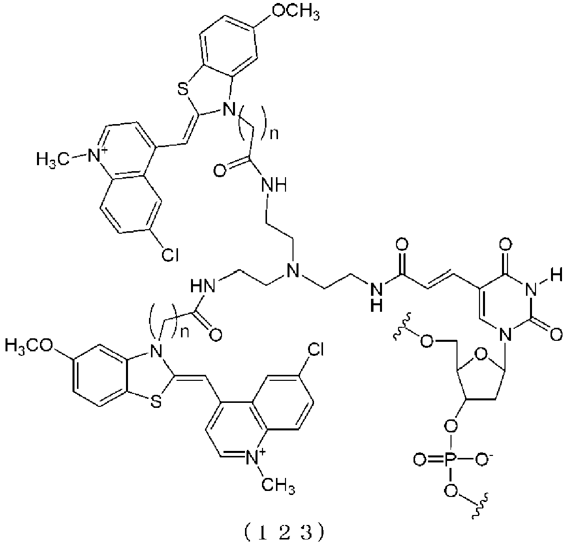

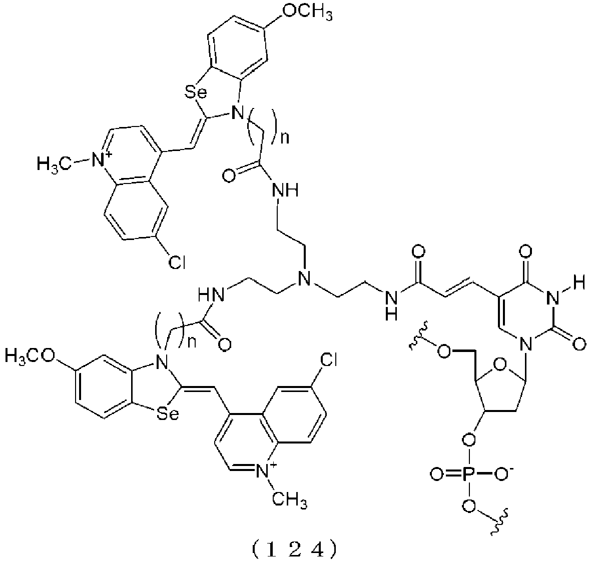

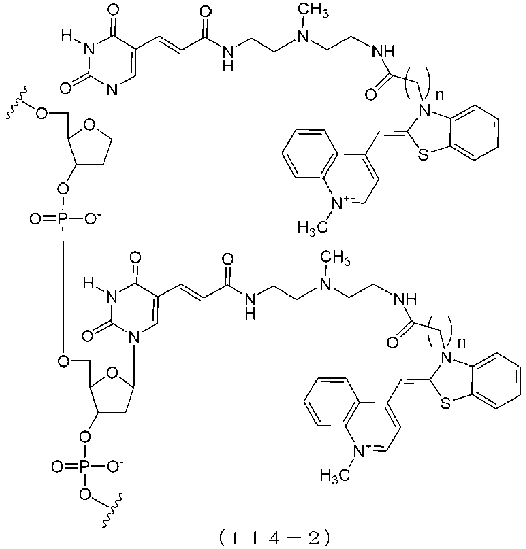

In the chemical formulae 106, 110, 113, 117, 120, 122, 123, 124, and 114-2, the linker length n preferably is a positive integer and in the range from 2 to 6.

The number of the labeled structures included in the Eprobe is not limited to particular values, and is, for example, about 1 to about 100, preferably about 1 to about 20. In the Eprobe, the site at which the labeled structure is included also is not limited to particular sites.

For the use of the intercalator, the detection step is preferably performed in the presence of the intercalator, for example. The intercalator is a fluorescent dye that develops color by intercalating with a double strand (double helix structure) formed by hybridization of the probe. The fluorescent dye is not limited to particularly dyes, and for example, SYBR (registered trademark) Green can be used.

The probe may be composed of, for example, any of a natural nucleotide residue, a non-nucleotide residue, a modified nucleotide residue, and a non-natural main backbone and may include one kind of them, two kinds of them, three kinds of them, or four kinds of them. The non-natural main backbone is not limited to particular backbones, and examples thereof include LNA, PNA, and a nucleic acid having a modified phosphodiester bond. The modified nucleotide residue is not limited to particular residues and is a phosphorothioated nucleotide residue, and the nucleotide residue may include a sulfur atom (S) or may be modified with the sulfur atom (S).

The basic backbone of the probe is not limited to particular backbones. Examples thereof include oligonucleotides, modified oligonucleotides, oligonucleosides, modified oligonucleosides, polynucleotides, modified polynucleotides, polynucleosides, modified polynucleosides, DNAs, modified DNAs, RNAs, modified RNAs, LNAs, PNAs (peptide nucleic acids), chimeric molecules thereof, and other structures. Furthermore, the basic backbone of each nucleic acid may be a natural one or an artificially synthesized one. In the case of the nucleic acid probe of the present invention, the nucleic acid is not particularly limited as long as it can provide base pairing, for example. In the case of a nucleic acid sample or a target nucleic acid sequence, the nucleic acid is not particularly limited as long as, for example, it serves as a template for synthesizing a complementary strand. Therefore the nucleic acid may be a nucleotide derivative, a part or the whole of which is formed of a completely artificial structure, for example. Examples of artificial bases that compose the nucleic acid include, but are not limited to, 2-amino-6-(N,N-dimethylamino)purine pyridin-2-one, 5-methylpyridin-2-one, 2-amino-6-(2-thienyl)purine, pyrrole-2-carbaldehyde, 9-Methylimidazo[(4,5)-b]pyridine, 5-iodo-2-oxo(1H)pyridine 2-oxo-(1H)pyridine, 2-amino-6-(2-thiazolyl)purine, and 7-(2-thienyl)-imidazo[4,5-b]pyridine.

In the present invention, the "nucleotide" may be either deoxynucleotide or ribonucleotide, for example, and the "oligonucleotide" and "polynucleotide" each may be composed of either one of deoxynucleotide and ribonucleotide or may contain both of them. In the probe, the number of bases that compose the nucleic acid is not limited to particular values. Generally, the term "nucleic acid" is synonymous with the term "polynucleotide". Generally, the term "oligonucleotide" is used as a term indicating a polynucleotide composed of a relatively small number of bases, among polynucleotides. In general, a polynucleotide of, for example, 2 to 100 nucleotides, more generally about 2 to 50 nucleotides is referred to as "oligonucleotide", but it is not limited by these numerical values. In the present invention, the term polynucleotide also should be interpreted to encompass, for example, polynucleotide and oligonucleotide, as well as artificially synthesized nucleic acids such as peptide nucleic acid, morpholine nucleic acid, methylphosphonate nucleic acid, and S-oligonucleic acid.

Generally, the peptide nucleic acid (PNA) has a structure in which a deoxyribose main chain of oligonucleotide has been substituted with a peptide main chain. Examples of the peptide main chain include a repeating unit of N-(2-aminoethyl)glycine bound with an amide bond. Examples of the base to be bound to the peptide main chain of PNA include, but not limited to: naturally-occurring bases such as thymine, cytosine, adenine, guanine, inosine, uracil, 5-methylcytosine, thiouracil, and 2,6-diaminopurine; and artificial bases such as bromothymine, azaadenine, and azaguanine.

Generally, LNA is a nucleic acid having two cyclic structures in which, in a sugar-phosphoric acid backbone, an oxygen atom in the 2'-position and a carbon atom in the 4'-position of ribose are bound to each other with methylene crosslinking. The double-stranded conformation changes by annealing of oligonucleotide containing LNA to DNA, thereby improving the thermal stability. LNA has a stronger binding affinity to a nucleic acid than common oligonucleotide. Thus, for example, depending on the conditions for designing the oligonucleotide, more reliable and stronger hybridization can be achieved.

The number of bases contained in the probe is not limited to particular numbers and is, for example, from about 5 to about 100, from about 6 to about 50, from about 6 to about 25.

As mentioned above, the detection step is a step of detecting association between the probe and the amplification product or dissociation of its assembly with a temperature change, a pH change, a concentration change of a denaturant, or a salt concentration change. It is widely known that single-stranded nucleic acids to be paired that are complementary in sequence to each other associate with each other (form a double strand), and the assembly is dissociated (the double strand is dissociated into single strands) with the various changes. In the detection step, the method for detecting the association or the dissociation of the assembly with a temperature change, a pH change, a concentration change of a denaturant, or a salt concentration change is not limited to particular methods.

In detection of the association with a temperature change in the detection step, signals at the respective temperatures are measured while reducing the temperature, for example. In the case where the signals are generated by the association, association between the probe and the amplification product occurs in order from relatively high Tm by reducing the temperature from the high temperature point to the low temperature point, and the signals are generated depending on the association, for example.

In detection of dissociation with a temperature change in the detection step, signals at the respective temperatures are measured while increasing the temperature, for example. In the case where the signals are generated by the association, the assembly between the probe and the amplification product is dissociated in order from relatively low Tm by increasing the temperature from the low temperature point to the high temperature point, and the signals disappear depending on the dissociation, for example.

The high temperature point and the low temperature point are not limited to particular temperature points and for example, can be determined, as appropriate, according to the probe to be used and the target sequence or the complementary sequence, which hybridizes with the probe. The high temperature point and the low temperature point can specifically be determined, as appropriate, according to the Tms thereof.

In the case where the present invention is applied to typing, the high temperature point and the low temperature point preferably satisfy, for example, the following condition (b1) or (b2):
(b1) the high temperature point is defined as any temperature point in a higher temperature region than the Tm in hybridization between "the probe" and "the target sequence having a nucleic acid mutation site to which the probe shows mismatch", and the low temperature point is defined as any temperature point in a lower temperature region than the Tm in hybridization between "the probe" and "the target sequence having a nucleic acid mutation site to which the probe shows fullmatch"; and
(b2) in the case where the probe shows mismatch to the nucleic acid mutation site and the probe shows different mismatch having a different Tm, the high temperature point is any temperature point in a higher temperature region than a relatively low Tm between the two Tms, and the lower temperature point is any temperature point in a lower temperature region than the relatively high Tm between the two Tms.

In detection of the association with a pH change in the detection step, fluorescence signals at the respective pHs are measured while reducing the pH, for example. In the case where the signals are generated by the association, association between the probe and the amplification product occurs in order from a relatively high Tm by reducing the pH from a high pH point to a low pH point, and the signals are generated depending on the association, for example. In detection of the dissociation of the assembly with a pH change in the detection step, light emission signals at the respective pHs are measured while increasing the pH, for example. In the case where the signals are generated by the association, the assembly between the probe and the amplification product is dissociated in order from a relatively low Tm by increasing the pH from a low pH point to a high pH point, and the signals disappear depending on the dissociation, for example.

A method for the detection in the present invention may, for example, further include a determination step of making an association curve or a melting curve (dissociation curve) on the basis of the detection results obtained in the detection step and determining the template nucleic acid on the basis of the curve. The determination of the template nucleic acid is not limited to particular determinations, and examples thereof include determination of the presence or absence of the template nucleic acid (qualitative analysis), determination of the amount of the template nucleic acid (quantitative analysis), and determination of the kind of a base at the nucleic acid mutation site in the template nucleic acid.

In the case where the detection results are obtained in the association with a temperature change in the detection step, the association curve is made in the determination step, for example. In the case where the detection results are obtained in the association with any of changes other than the temperature change (e.g., a pH change, a concentration change of a denaturant, a salt concentration change), the temperature change can be read as any of these changes in the following description of the association curve.

A method for making the association curve is not limited to particular methods. The association curve can be made on the basis of, for example, a change from a signal disappearance state (signal non-generation state) caused by the probe that does not associate with the amplification product to a signal generation state caused by association (formation of a double strand) between the probe and the amplification product. Specifically, for example, differential values (dF/dT) of variations in signal and time are determined from measurement results of signals at the respective temperatures, obtained in the detection step, and a curve showing a correlation between the temperatures and the differential values (dFT/dT) is made as the association curve. The association curve may be, for example, a positive primary differential curve (dF/dT) or a negative primary differential curve (-dF/dT) and is preferably a positive primary differential curve because the generation of the signals can be determined by a peak in an inverted U-shaped curve. The association curve hereinafter indicates a positive primary differential curve in the specification. The present invention, however, is by no means limited thereby, and the association curve can be read as a U-shaped curve in a negative primary differential curve (-dF/dT), for example.

In the case where detection results are obtained in the dissociation of the assembly with a temperature change in the detection step, the melting curve (dissociation curve) is made in the determination step, for example. In the case where the detection results are obtained in the association with any of changes other than the temperature change (e.g., a pH change, a concentration change of a denaturant, a salt concentration change), the temperature can be read as pH or the like in the following description of the melting curve.

A method for making the melting curve is not limited to particular methods. The melting curve can be made on the basis of, for example, a change from a signal generation state caused by association (formation of a double strand) between the probe and the amplification product to a signal disappearance state (signal non-generation state) caused by the dissociation of the assembly (dissociation of the double strand). Specifically, for example, differential values (dF/dT) of variations in signal and time are determined from measurement results of signals at the respective temperatures, obtained in the detection step, and a curve showing a correlation between the temperatures and the differential values (dFT/dT) is made as the melting curve. The melting curve may be, for example, a positive primary differential curve (dF/dT) or a negative primary differential curve (-dF/dT) and is preferably a negative primary differential curve because the disappearance of the signals can be determined by a peak in an inverted U-shaped curve. The melting curve hereinafter indicates a negative primary differential curve in the specification. The present invention, however, is by no means limited thereby, and the melting curve can be read as a U-shaped curve in a positive primary differential curve (dF/dT), for example.

In the determination step, a peak in the association curve or the melting curve is checked, for example. In qualitative analysis or quantitative analysis of the template nucleic acid as the analysis of the template nucleic acid, when a peak is found at the Tm of the target sequence or the complementary sequence with the probe, it can be determined that the template nucleic acid is present, and when no peak is found at the Tm, it can be determined that the template nucleic acid is not present, for example. Moreover, the amount of the template nucleic acid can also be determined on the basis of the size of the peak, for example. In typing as the analysis of the template nucleic acid, whether the nucleic acid mutation site is of, for example, a wild type or a mutant type and whether plural mutant-type nucleic acid mutation sites are homozygous or heterozygous can be determined on the basis of the presence or absence of a peak at each Tm based on the base at the nucleic acid mutation site, for example. Each of the association curve and the melting curve is hereinafter referred to as an analysis curve in the description, unless otherwise shown.

By appropriately selecting any of the conditions (A) to (E), the present invention can exert, for example, a desired effect among the first effect of reducing background noise, the second effect of sharpening a peak, the third effect of increasing the distance between heterozygous peaks, and the fourth effect of improving the degree of separation of the heterozygous peaks.

The following describes the present invention with reference to embodiments. The following embodiments use a probe (probe for Tseq) to be hybridized with the target sequence as an example, unless otherwise shown. The present invention, however, is by no means limited thereto, and the probe for Cseq can be used as a substitute for the probe for Tseq.

### Embodiment 1A

The present embodiment satisfies the condition (A) and specifically uses, as the primer in the amplification step, a turnback primer that also serves as the probe in the detection step. Hereinafter, the turnback primer is also referred to as a TB primer.

In the analysis method, (A) a primer set is used in the amplification step; the primer set is a combination of a first primer for a polymerase that performs priming at the 3' end of a primer to synthesize the target sequence and a second primer for the polymerase to synthesize the complementary strand; between the first primer and the second primer, a primer that initiates synthesis of a sequence to be hybridized with the probe is a turnback primer including a primer region and a probe region being directly or indirectly linked to the 5' end of the primer region, and the other primer is any primer having an ability of causing the polymerase to perform priming at the 3' end; in the amplification step, the turnback primer extends the target sequence or the complementary sequence from the primer region; and in the detection step, the turnback primer also serves as the probe, and association between the probe region and the target sequence or the complementary sequence, occurred by turning the probe region of the turnback primer in a molecule of the amplification product back toward the extended target sequence or the extended complementary sequence is detected.

In the present embodiment, either one of the first primer or the second primer is a TB primer, and the other is a non-TB primer. The present embodiment uses a probe (probe for Tseq) to be hybridized with the target sequence as an example. The present embodiment is thus described below with reference to the first primer that synthesizes the target sequence as an example of the TB primer. In addition, hereinafter, the template nucleic acid is referred to as a template strand, and the complementary strand to the template nucleic acid is referred to as a complementary strand.

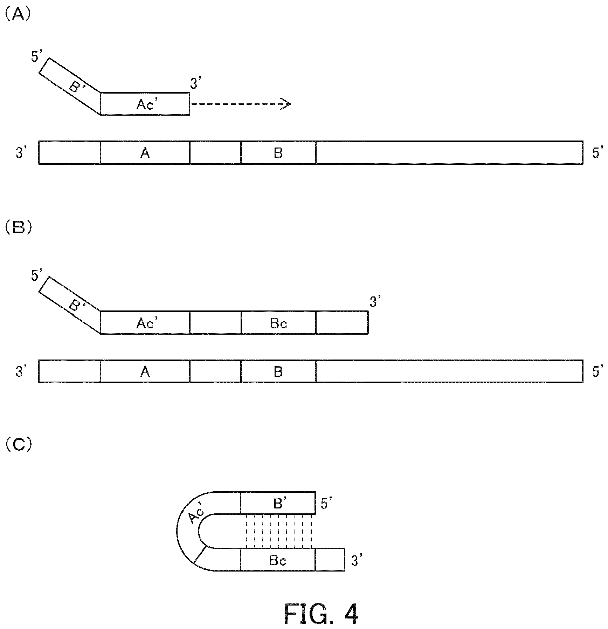

The TB primer is described below with reference to FIG. 4. FIG. 4 shows schematic views illustrating an example TB primer. In FIG. 4, (A) is a schematic view illustrating a relationship between a TB primer as the first primer that synthesizes the target sequence and the complementary strand thereto, (B) is a schematic view illustrating an extension of the TB primer in the amplification step, and (C) is a schematic view illustrating turning back of the TB primer in the detection step.

As shown in (A) in FIG. 4, the TB primer includes a primer region (Ac') and a probe region (B') linked to the 5' end of the primer region (Ac'). The probe region (B') is set to be the same sequence as any region (B) in the complementary strand. The primer region (Ac') is set to be a sequence to be hybridized with any region (A) on the downstream side of the region (B) in the complementary strand.

When the TB primer is used in the amplification step, the primer region (Ac') of the TB primer anneals to the region (A) of the complementary strand, and the extension reaction is initiated from the 3' end of the TB primer. The TB primer is on the 5' side of the obtained extended strand, and an extended region is on the 3' side of the same. The extended region includes a complementary region (Bc) to the region (B).

Intermolecular association of the extended strand then occurs in the detection step. That is, the probe region (B') in the extended strand is the same sequence as the region (B) in the complementary strand and thus is complementary to the complmentary region (Bc) in the extended strand. The extended strand thus is intramolecularly turned back, and intramolecular association between the probe region (B') and the complementary region (Bc) occurs. In the detection step, the intramolecular association of the extended strand or dissociation of its assembly is detected.

The present embodiment using the TB primer as described above exerts, for example, the following effects.

First, the present embodiment exerts the first effect, i.e., an effect of preventing nonspecific detection, and this effect is also referred to as an effect of improving detection specificity. For example, this effect leads to a reduction in background noise that is caused by the nonspecific detection. In the case where TB primer is used, the probe region (B') and the complementary region (Bc) are both present in a molecule of the extended strand. Contact efficiency of both of the probe region (B') and the complementary region (Bc) according to the Brownian motion is thus really high compared with the case where the probe and the extended strand are different molecules. The probe region (B') and the complementary region (Bc) can thus be efficiently hybridized with each other. If the TB primer anneals to a region other than the region (A) and nonspecifically amplifies a non-target sequence that is different from the target sequence, the complementary region (Bc) to be hybridized with the probe region (B') is not present in the extended strand. Thus, if nonspecific amplification occurs, the extended strand is not intramolecularly turned back. Nonspecific association of the probe region (B') thus can be prevented. That is, it can be said that the intramolecular association of the extended strand occurs only when the TB primer anneals to the predetermined region (A) and synthesizes a target sequence, and the association or dissociation of the assembly can be detected.

The present embodiment further exerts the second effect, i.e., an effect of sharpening a peak in the analysis curve. The sharpening of the peak means, for example, reducing the half width of the peak.

The present embodiment further exerts the fourth effect, i.e., an effect of improving the degree of separation of heterozygous peaks in typing.

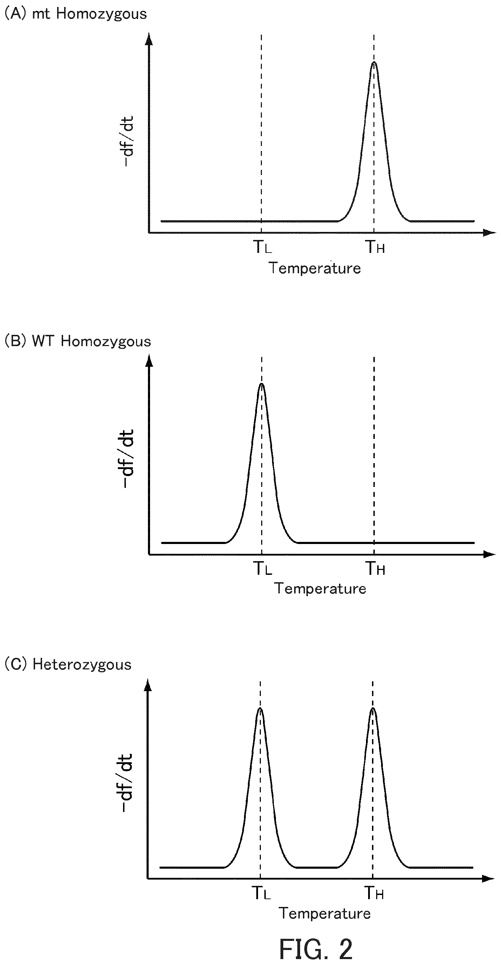

The first, second, and fourth effects of the present embodiment result in obtaining peaks shown in FIG. 2, for example. FIG. 2 shows drawings illustrating example analysis curves (e.g., melting curves) obtained in the present embodiment and shows an example of using a probe that is fullmatch to a mutant-type nucleic acid mutation site and mismatch to a wild-type nucleic acid mutation site. In FIG. 2, (A) shows an analysis curve of a homozygous mutant-type target sequence, and a sharp peak is obtained at a temperature (T_{H}) for fullmatch, and background noise is reduced. In FIG. 2, (B) shows an analysis curve of a homozygous wild-type target sequence, a sharp peak is obtained at a temperature (T_{L}) for mismatch, and background noise is reduced. In FIG. 2, (C) shows an analysis curve of heterozygous target sequence, and the respective sharp peaks are obtained at a temperature (T_{H}) and a temperature (T_{L}), and background noise is reduced.

The present embodiment may further satisfy the following conditions.
(A-1) The present embodiment further includes, for example, a step of performing the amplification step in the presence of a nickase or adding a nickase after the amplification step to design a nickase recognition sequence in "the turnback primer or a nucleic acid strand synthesized of the turnback primer" and nick a complementary strand to "the turnback primer or the nucleic acid strand synthesized of the turnback primer". The nickase is, for example, an enzyme for nicking a base sequence and is not limited to particular enzymes, and known enzymes can be used.
   The site (position) of the TB primer and the nucleic acid strand synthesized of the TB primer, at which the nickase recognition sequence is designed, is not limited to particular sites.
   The present embodiment can further improve the first effect, i.e., the effect of preventing nonspecific detection by satisfying this condition for the following reasons. In the amplification step, synthesis is performed in both directions using the first primer and the second primer. Thus, for example, in the case of using the TB primer as the first primer that synthesizes the target sequence, the second primer anneals to the nucleic acid strand (extended strand) obtained of the TB primer, and a nucleic acid strand (extended strand) including a complementary region to the TB primer is synthesized in some cases. However, a nucleic acid strand obtained of the second primer includes a complementary region to the TB primer. The nucleic acid strand thus may hybridize, in the complementary region, with a probe region of the TB primer. However, in the present embodiment, the nickase recognition sequence is designed in any site of "the TB primer and a nucleic acid strand synthesizes of the TB primer". The complementary strand to "the TB primer and the nucleic acid strand synthesized of the TB primer" can be nicked. The nicking causes a nucleic acid strand synthesized of the second primer to be short. The Tm of the TB primer thus can be sufficiently low. The influence of nonspecific hybridization between the extended strand from the second primer and the TB primer on detection thus can be sufficiently prevented.
(A-2) In the TB primer of the present embodiment, the primer region and the probe region may be directly or indirectly linked to each other, for example. The former case is a form of having no intervening sequence between the primer region and the probe region. The latter case is a form of having an intervening sequence between the primer region and the probe region and specifically a form where the probe region is indirectly linked to the 5' end of the primer region via an intervening sequence, for example. In the present embodiment, the TB primer preferably has an intervening sequence. The TB primer having an intervening sequence can be turned back further easily, for example, and the first, second, and fourth effects in the present embodiment can further be improved. In the TB primer having an intervening sequence, the intervening sequence preferably has a nickase recognition sequence.
(A-3) In the present embodiment, the TB primer preferably satisfies the condition (a1), (a1), (a2), or (a2'), for example. The TB primer satisfying any of the conditions can be more easily turned back, for example. The first, the second, and the fourth effects of the present embodiment thus can further be improved.
(a1) In the case where the first primer is the turnback primer and an intervening sequence is absent between the primer region (Ac') and the probe region (B'), (X - Y)/(X) is in the range from -1 to 1, assuming that the number of bases of the primer region (Ac') is X, and the number of bases of a region sandwiched between a region (A) to be hybridized with the primer region (Ac') and a region (B) to be hybridized with the probe (B') in the complementary sequence is Y
(a1') In the case where the first primer is the turnback primer, and an intervening sequence is present between the primer region (Ac') and the probe region (B'), [X - (Y - Y')]/X is in the range from -1 to 1, assuming that the number of bases of the primer region (Ac') is X, the number of bases of a region sandwiched between a region (A) to be hybridized with the primer region (Ac') and a region (B) to be hybridized with the probe (B') is Y, and the number of bases of the intervening sequence is Y'.
(a2) In the case where the second primer is the turnback primer, and an intervening sequence is absent between the primer region (cAc') and the probe region (cB'), (X - Y) /(X) is in the range from -1 to 1, assuming that the number of bases of the primer region (cAc') is X, and the number of bases of a region sandwiched between a region (cA) to be hybridized with the primer region (cAc') and a region (cB) to be hybridized with the probe (cB') in the target sequence is Y
(a2') In the case where the second primer is the turnback primer, and an intervening sequence is present between the primer region (cAc') and the probe region (cB'), [X - (Y - Y')]/X is in the range from -1 to 1, assuming that the number of bases of the primer region (cAc') is X, the number of bases of a region sandwiched between a region (cA) to be hybridized with the primer region (cAc') and a region (cB) to be hybridized with the probe (cB') in the target sequence is Y, and the number of bases in the intervening sequence is Y'.

In the absence of an intervening sequence as described in the condition (a1) or (a2), the lower limit of (X - Y)/X is designed to be, for example, -1.00 or more, 0.00 or more, 0.05 or more, 0.10 or more, the upper limit of (X - Y)/X is designed to be, for example, 1.00 or less, 0.75 or less, 0.50 or less, 0.25 or less, the lower limit of (X + Y) is designed to be, for example, 15 or more, 20 or more, 30 or more, and the upper limit of (X + Y) is designed to be, for example, 50 or less, 48 or less, 42 or less.

In the presence of an intervening sequence (the number of bases: Y') as described in the condition (a1') or (a2'), the lower limit of {X - (Y - Y')}/X is designed to be, for example, -1.00 or more, 0.00 or more, 0.05 or more, 0.10 or more, the upper limit of {X - (Y - Y')}/X is designed to be, for example, 1.00 or less, 0.75 or less, 0.50 or less, 0.25 or less, the lower limit of (X + Y + Y') is designed to be, for example, 15 or more, 20 or more, 30 or more, and the upper limit of (X + Y + Y') is designed to be, for example, 100 or less, 75 or less, 50 or less.

The length of the TB primer is not limited to particular lengths, and the full length thereof is, for example, from 15 to 100 nucleotides, from 20 to 60 nucleotides.

In the case where the first primer is the TB primer, the length of each of the primer region (AC') and the probe region (B') is, for example, from 5 to 50 nucleotides, from 7 to 30 nucleotides. In the case where the second primer is the TB primer, the length of each of the primer region (cAC') and the probe region (cB') is, for example, from 5 to 50 nucleotides, from 7 to 30 nucleotides.

### Embodiment 1B

The present embodiment satisfies the condition (B) and specifically is a method for analyzing a template nucleic acid, wherein the detection step includes a step of detecting association between the probe and the amplification product with a temperature change while changing a temperature from a high temperature point to a low temperature point.

The present embodiment can exert, for example, the first effect, i.e., an effect of preventing nonspecific detection, and this effect can be an effect of improving detection specificity. This effect, for example, leads to a reduction in background noise that is caused by the nonspecific detection.

Single-stranded nucleic acids to be paired that are complementary to each other associate with each other in a lower temperature region than the Tm, and the assembly is dissociated at about Tm. Thus, a lower temperature is set as an initial temperature to cause the single stranded nucleic acids to be paired to be in the associated state, the temperature is increased to a high temperature point to dissociate the assembly, and the dissociation is then detected in the method for analyzing a template nucleic acid using a probe. The Tm differs depending on, for example, the sequences and lengths of the single-stranded nucleic acids to be paired, and the higher the specificity of the single-stranded nucleic acids to each other is, the higher the Tm is. Thus, in a temperature change from a low temperature point to a high temperature point, association having a low Tm and low specificity, i.e., nonspecific association occurs. Then, in the increase in temperature from the low temperature point to the high temperature point, dissociation of this nonspecific assembly is also detected, and as a result, background is increased. In contrast, in the present embodiment, the high temperature point is set as an initial temperature, and the temperature is reduced to the low temperature point. The probe and the amplification product are thus in the dissociated state at the initial temperature, and association occurs with a temperature reduction in order from low Tm. The detection step detects this association, and the above-mentioned increase in background noise caused by nonspecific association thus can be prevented.

The present embodiment can further exert the second effect, i.e., an effect of sharpening a peak in the analysis curve. The sharpening of the peak means, for example, reducing the half width of the peak.

The present embodiment can further exert the fourth effect, i.e., an effect of improving the degree of separation of heterozygous peaks in typing.

In the case of heterozygous template nucleic acid, the probe is associated with both of a target sequence having a fullmatch nucleic acid mutation site to the probe and a target sequence having a mismatch nucleic acid mutation site to the probe. The assembly is dissociated at a higher temperature in the former case and at a lower temperature in the latter case. Thus, in the typing, whether the template nucleic acid is heterozygous or homozygous can be detected by detecting the dissociation at the higher temperature and the lower temperature. However, the probe prone to bind preferentially to the target sequence having a fullmatch nucleic acid mutation site to the probe (hereinafter referred to as a fullmatch target nucleic acid) compared with the target sequence having a mismatch nucleic acid mutation site to the probe (hereinafter referred to as a mismatch target nucleic acid). The probe thus cannot associate with the mismatch target sequence in some cases. In this case, if the template nucleic acid is heterozygous, the fullmatch target sequence may only be detected. In contrast, if the template nucleic acid is heterozygous, the ratio between the amount of the amplification product and the amount of the probe of 1 : 1 allows the probe to associate with both of the mismatch target sequence and the fullmatch target sequence. Furthermore, in the present embodiment, a sequence to be hybridized with the probe is synthesized using a TB primer including a primer region and a probe region. The ratio between the amount of the probe (probe region) and the amount of the amplification product in the detection step is thus theoretically 1 : 1. The present embodiment thus can detect both of the fullmatch target sequence and the mismatch target sequence and can improve the degree of separation of heterozygous peaks if the template nucleic acid is heterozygous template nucleic acid.

The high temperature point and the low temperature point are not limited to particular points and can be, for example, determined on the basis of, the length and the sequence of the probe, and the length and the sequence of the target sequence to be hybridized with the probe or the complementary sequence thereto.

In the typing as analysis of the template nucleic acid, the high temperature point and the low temperature point preferably satisfy the condition (b1) or (b2), for example:
(b1) the high temperature point is defined as any temperature point in a higher temperature region than the Tm in hybridization between "the probe" and "the target sequence having a nucleic acid mutation site to which the probe shows mismatch", and the low temperature point is defined as any temperature point in a lower temperature region than the Tm in hybridization between "the probe" and "the target sequence having a nucleic acid mutation site to which the probe shows fullmatch"; and
(b2) in the case where the probe shows mismatch to the nucleic acid mutation site and the probe shows different mismatch having a different Tm, the high temperature point is any temperature point in a higher temperature region than a relatively low Tm between the two Tms, and the lower temperature point is any temperature point in a lower temperature region than the relatively high Tm between the two Tms.

### Embodiment 1C

The present embodiment satisfies the condition (C) and specifically uses the first primer and the second primer at a predetermined ratio.

A method for analyzing a template nucleic acid satisfies the condition (C): in the amplification step, a primer set is used, the primer set is a combination of a first primer that performs priming of a polymerase that synthesizes the target sequence and a second primer that performs priming of a polymerase that synthesizes the complementary strand, and between the first primer and the second primer, a primer that performs priming of a polymerase that synthesizes a sequence to be hybridized with the probe or the probe region in the condition (A) is used in an amount higher than the other primer.

The present embodiment uses a higher amount of a primer (e.g., the first primer) that synthesizes a sequence (e.g., the target sequence) to be hybridized with the probe than the other primer (e.g., the second primer) and thus selectively increase an extended strand from the former primer, i.e., the sequence to be hybridized with the probe. Accordingly, the present embodiment exerts, for example, the first effect, i.e., an effect of preventing nonspecific detection, and a background can be reduced.

The present invention further exerts the second effect, i.e., an effect of sharpening a peak in an analysis curve. The sharpening the peak means, for example, reducing the half width of the peak.

The present embodiment further exerts the fourth effect, i.e., an effect of improving the degree of separation of heterozygous peaks in typing.

The ratio between a primer (M) that synthesizes the sequence to be hybridized with the probe and the other primer (S) to be used is not limited to particular ratios. X is, for example, in the range of 1 < X ≤ 10⁶, assuming that the concentration of the primer (M) is higher than that of the other primer (S), and the molar ratio (M : S) is X : 1. X is, for example, X ≤ 10³, X ≤ 10², X ≤ 10, 2 ≤ X ≤ 9, 2 ≤ X ≤ 8, 2 ≤ X ≤ 7, 3 ≤ X ≤ 6.

### Embodiment 1D

The present embodiment satisfies the condition (D) and, the probe is, for example, an Eprobe.

A method for analyzing a template nucleic acid satisfies the condition (D) in the probe, the length of a region to be hybridized with the target sequence or the complementary sequence is 6 to 100 nucleotides.

The length of the region is, for example, from 6 to 40 nucleotides, from 6 to 25 nucleotides, from 9 to 20 nucleotides, from 10 to 18 nucleotides.

The present embodiment exerts the third effect, i.e., an effect of increasing the distance between heterozygous peaks in typing.

The present embodiment further exerts the fourth effect, i.e., an effect of improving the degree of separation of heterozygous peaks in typing.

The Eprobe shows a higher Tm than a normal probe having the same length as the Eprobe and is not bound to a fluorescent atomic group that exhibits the excitonic effect. The Eprobe thus can be set to have a shorter base length than the normal probe to cause the Eprobe to show the same Tm as the normal probe. Assuming that the base length of the normal probe is 20 nucleotides, and the base length of the Eprobe is 13 nucleotides, the relationship between the probe and the target sequence is represented by 20 nucleotides/20 nucleotides (= 1) in the case where the normal probe is fullmatch to the target sequence, and the relationship is represented by 19 nucleotides/20 nucleotides (= 0.95) in the case where the normal probe is mismatch to the target sequence. On the other hand, the relationship is represented by 13 nucleotides/13 nucleotides (= 1) in the case where the Eprobe is fullmatch to the target sequence, and the relationship is represented by 12 nucleotides/13 nucleotides (= 0.923) in the case where the Eprobe is mismatch to the target sequence. That is, the Eprobe shows a larger difference between the fullmatch and the mismatch. Thus, for example, if the template nucleic acid is heterozygous, the difference, i.e., the distance between a wild-type peak and a mutant-type peak can be increased, and the degree of separation of both of the peaks can be improved.

### Embodiment 1E

The present embodiment satisfies the condition (E), and specifically, the primer set is designed to be close to a region to be hybridized with the probe in the target sequence or the complementary sequence.

A method for analyzing a template nucleic acid satisfies the condition (E) the primer set is a combination of a first primer that performs priming of a polymerase that synthesizes the target sequence and a second primer that performs priming of a polymerase that synthesizes the complementary sequence, the first primer has the same sequence as an upstream region at any site of the template nucleic acid, the second primer has a complementary sequence to a downstream region at any site of the template nucleic acid, and the analysis method further satisfies the condition (e1) or (e2): (e1) the probe hybridizes with the target sequence, the 3' end of the upstream region in the template nucleic acid is positioned in the vicinity of the upstream side of the 5' end of a region to be hybridized with the probe in the template sequence, and the 5' end of the downstream region in the template nucleic acid is positioned in the vicinity of the downstream side of the 3' end of a region to be hybridized with the probe in the template sequence; and (e2) the probe hybridizes with the complementary sequence, the 3' end of the upstream region in the template nucleic acid is positioned in the vicinity of the upstream side of the 5' end of a region that overlaps in sequence with the probe in the template nucleic acid, and the 5' end of the downstream region in the template nucleic acid is positioned in the vicinity of the downstream side of the 3' end of a region that overlaps in sequence with the probe in the template nucleic acid.

When the probe hybridizes with the target sequence, association between the probe and a relatively short target sequence shows a higher Tm and higher specificity than association between the probe and a relatively long target sequence. The primer set used in the present embodiment is set to anneal to the vicinity of a region to be hybridized with the probe in the target sequence or the complementary sequence as mentioned above. A shorter target sequence or a shorter complementary sequence thus can be obtained in the present embodiment by the synthesis using the primer set. The present embodiment thus allows association having higher specificity. The present embodiment therefore exerts, for example, the first effect, i.e., an effect of preventing nonspecific detection, and a background can be reduced.

The present embodiment further exerts the second effect, i.e., an effect of sharpening a peak in the analysis curve.

### Embodiment 1F

The analysis method of the present invention may be any one of the combinations of two or more or all of the conditions in the embodiments 1A to 1E. The combinations are as follows:
the combination of the conditions (A) and (B);
the combination of the conditions (A), (B), and (C);
the combination of the conditions (A), (B), (C), and (D);
the combination of the conditions (A), (B), (C), (D), and (E);
the combination of the conditions (A) and (C);
the combination of the conditions (A), (C), and (D);
the combination of the conditions (A), (C), (D), and (E);
the combination of the conditions (A) and (D);
the combination of the conditions (A), (D), and (E);
the combination of the conditions (A) and (E);
the combination of the conditions (B) and (C);
the combination of the conditions (B), (C), and (D);
the combination of the conditions (B), (C), (D), and (E);
the combination of the conditions (B) and (D);
the combination of the conditions (B), (D), and (E); and
the combination of the conditions (B) and (E).

### Embodiment 1G

The analysis method of the present invention preferably further satisfies the following conditions, for example. The following conditions can be applied to each of the embodiments 1A to 1F, for example.

The detection step is, for example, preferably performed in the presence of any of the above-mentioned additives (for example, DMSO). In the presence of the additive, an increase in background in a low temperature region can be prevented (the first effect), or a peak can be sharpened (the second effect), for example.

The probe contains, for example, at least one of the non-nucleotide residue, the modified nucleotide residue, and the non-natural main backbone. The non-nucleotide residue, the modified nucleotide residue, and the non-natural main backbone are, for example, as mentioned above. The probe containing any of these can increases the Tm of the probe. The present embodiment thus allows, for example, a peak to be sharpened (the second effect) and the distance between heterozygous peaks to be increased.

The probe is, for example, an FRET probe including at least two fluorescent atomic group pairs that exhibit an excitonic effect or an FRET probe including at least one fluorescent atomic group pair that exhibits an excitonic effect and a dye that exhibits no excitonic effect, and the two fluorescent atomic group pairs are bound to the same base in the probe via linkers or the respective bases that are adjacent to each other and are arranged to exhibit an FRET effect in the probe. In the probe, for example, when one of the two pairs is excited and emits fluorescence, the other pair is excited by the fluorescence and emits different fluorescence. The probe thus can be an FRET probe. The probe can increase the Tm of the probe, which results in improvement of S/N. The present embodiment thus allows, for example, an increase in background noise in a low temperature region to be prevented (the first effect), a peak to be sharpened (the second effect), and the distance between heterozygous peaks in typing to be increased (the third effect).

In the FRET probe, for example, an emission peak wavelength of one (hereinafter referred to as the "fluorescent atomic group pair A") of the two fluorescent atomic group pairs is lower than the other (hereinafter referred to as the "fluorescent atomic group pair B") (characteristic A), and the fluorescent atomic group pairs A and B exhibit an FRET effect (characteristic B).

In the FRET probe, for example, a base having the fluorescent atomic group pair A and a base having the fluorescent atomic group pair B are contained in the probe at a distance at which the fluorescent atomic group pairs A and B exhibit an FRET effect.

In the FRET probe, the distance between the base having the fluorescent atomic group pair A and the base having the fluorescent atomic group pair B is, for example, from 1 to 11 bases.

In the FRET probe, the base having the fluorescent atomic group pair that exhibits an excitonic effect has, for example, as mentioned above, a structure represented by the formula (16), (16b), (17), or (17b).

The base length of the FRET probe is, for example, in the range from 4 to 100 nucleotides, from 10 to 50 nucleotides, from 10 to 40 nucleotides, from 10 to 30 nucleotides. The base length is selected, as appropriate, according to the purpose of the use thereof, for example. The number of fluorescent atomic group pairs that exhibit an excitonic effect in the FRET probe is not limited to particular values and is at least two and can specifically be two or three or more. In order to exhibit an FRET effect, the number of fluorescent atomic group pairs that exhibit an excitonic effect is practically only necessary to be two, for example. However, considering the purpose of the use of the fluorescent atomic groups, the kind of the same, the distance between the same, and the extent of the FRET effect, the number may be three and may also be four or more, for example.

The excitonic effect, for example, suppresses the fluorescence intensity in a single strand state, thereby allowing a double helix structure to be detected further effectively. The excitonic effect (exciton coupling) is an effect in which, for example, a plurality of dyes aggregate in parallel to form an H-aggregate and thus hardly exhibit fluorescence emission. Conceivably, this effect is obtained as follows. That is, the excitation state of the dye is split into two energy levels by Davydov splitting, excitation to the higher energy level and then internal conversion into the lower energy level occur, and thereby the emission is thermodynamically forbidden. However, these descriptions do not limit the present invention by any means. The possible occurrence of the excitonic effect can be confirmed by the appearance of the absorption band of the dyes that have formed the H-aggregate, in a shorter wavelength as compared to the absorption band of a single dye. Examples of the dyes that exhibit such an effect include thiazole orange and derivatives thereof, thiazole pink and derivatives thereof, oxazole yellow and derivatives thereof, cyanine and derivatives thereof, hemicyanine and derivatives thereof, and methyl red and derivatives thereof, as well as dye groups generally referred to as cyanine dyes and azo dyes.

These dyes are, for example, easily bound to a DNA-DNA double strand and a DNA-RNA double strand each of which forms a double helix or a double strand formed of an artificial nucleic acid such as phosphorothioate nucleic acid, PNA (peptide nucleic acid), or locked nucleic acid (LNA) (BNA) with DNA or RNA by intercalation. When a plurality of such dyes have been introduced into a single-stranded nucleic acid, strong quenching occurs in the general single strand state (for example, the state of only a probe before hybridization). When the single-stranded nucleic acid hybridizes with a target DNA or RNA, the aggregate is resolved, and the dyes are individually intercalated into the double strand. At that time, there is no electronic interaction between the dyes, thereby exhibiting no excitonic effect and exhibiting intense fluorescence emission. The absorption band of the dyes at that time is the same as the absorption band of the single dye, and this demonstrates that the excitonic effect is not exhibited between the dyes. When the dyes are intercalated into a double strand, the twist on the structure originally in the dyes is resolved, and thus, the fluorescence emission becomes further intense.

### Characteristic (a)

The emission peak wavelength(s) of one of the fluorescent atomic group pairs (the fluorescent atomic group pair A) is shorter than an excitation peak wavelength(s) of the other fluorescent atomic group pair (the fluorescent atomic group pair B). The emission peak wavelength means a peak wavelength of emission spectrum generated at the time when the fluorescent atomic group pair A is irradiated with excitation light and changes according to the types of fluorescent atomic groups of the fluorescent atomic group pair A. The excitation peak wavelength means a peak wavelength of spectrum of excitation light that can be absorbed by the fluorescent atomic group pair B and changes according to the types of fluorescent atomic groups of the fluorescent atomic group pair B. The emission peak wavelength(s) of the fluorescent atomic group pair A and the excitation peak wavelength(s) of the fluorescent atomic group pair B are not limited. Note here that when the FRET probe is used for detection of a fluorescent label, fluorescence is, for example, emitted from the fluorescent atomic groups of the fluorescent atomic group pair B, and thus, fluorescent atomic groups of the fluorescent atomic group pair B having emission intensity and a wavelength(s) that are suitable for detection are selected, and considering the excitation peak wavelength(s) of the fluorescent atomic groups of the fluorescent atomic group pair B, fluorescent atomic groups of the fluorescent atomic group pair A can be selected. The relationship between the emission peak wavelength(s) of the fluorescent atomic groups of the fluorescent atomic group pair A and the excitation peak wavelength(s) of the fluorescent atomic groups of the fluorescent atomic group pair B can be, for example, determined considering the FRET effect obtained between them. The two fluorescent atomic groups in the fluorescent atomic group pair A may be identical to or different from each other, and the two fluorescent atomic groups in the fluorescent atomic group pair B may also be identical to or different from each other. When the two fluorescent atomic groups in the fluorescent atomic group pair A or the two fluorescent atomic groups in the fluorescent atomic group pair B are different from each other, the emission peak wavelength of at least one of the two fluorescent atomic groups of the fluorescent atomic group pair A is shorter than the excitation peak wavelength of at least one of the two fluorescent atomic groups of the fluorescent atomic group pair B. It is preferred that the emission peak wavelengths of both of the fluorescent atomic groups of the fluorescent atomic group pair A are shorter than the excitation peak wavelengths of both of fluorescent atomic groups of the fluorescent atomic group pair B.

### Characteristic (b)

The fluorescent atomic group pairs A and B exhibit the FRET effect. The FRET effect is also called fluorescence resonance energy transfer and is a phenomenon where the excitation energy between adjacent two chromophores is not converted into an electromagnetic wave and is directly transferred by the resonance of electrons. The energy is transferred, by energy of light absorbed by one of the chromophores (donor), to the other chromophore (receptor), and when the receptor is a fluorescent molecule, fluorescence is emitted from the receptor. In the labeled single-stranded nucleic acid of the present invention, the fluorescent atomic group pair A having an emission peak wavelength that is shorter than the excitation peak wavelength of the fluorescent atomic group pair B is arranged so as to exhibit the FRET effect with the fluorescent atomic group pair B. The arrangement by which the fluorescent atomic group pairs A and B exhibit the FRET effect is, for example, an arrangement in the case where a base having the fluorescent atomic group pair A and a base having the fluorescent atomic group pair B are contained in the labeled single-stranded nucleic acid at a distance at which the fluorescent atomic group pairs A and B have the FRET effect. The distance (base length) at which the fluorescent atomic group pairs A and B have the FRET effect is, although it differs according to the types and combinations of fluorescent atomic groups of the fluorescent atomic group pairs A and B, for example, 1 to 11 bases, preferably 2 to 8 bases, more preferably 2 to 7 bases, yet more preferably 2 to 6 bases, yet more preferably 2 to 5 bases, yet more preferably 3 to 5 bases. The distance of one base means that one nucleic acid having no fluorescent atomic group is present between the fluorescent atomic group pairs A and B. Examples of the combinations of the fluorescent atomic groups of the fluorescent atomic group pairs A and B include a combination of thiazole orange (D514) and thiazole pink (D570) or D640 and a combination of D436 and thiazole orange, thiazole pink, or D640.

In addition to the characteristics (a) and (b), it is preferred that the FRET probe is optionally characterized in that the fluorescent atomic group pairs A and B are positioned at any of the bases that is at least two bases inward from each end of the FRET probe, for example. By satisfying this characteristic, both of the excitonic effect and the FRET effect can be exhibited, for example.

This application claims priority from Japanese Patent Application No. 2015-041813 filed on March 3, 2015, the entire subject matters of which are incorporated herein by reference.

### Industrial Applicability

The present invention can analyze a template nucleic acid with higher accuracy.

## Claims

1. A method for analyzing a template nucleic acid, comprising:
an amplification step of amplifying a target sequence in the template nucleic acid and a complementary sequence to the target sequence; and
a detection step of detecting association between a probe and an amplification product obtained in the amplification step or dissociation of an assembly between the probe and the amplification product, with a temperature change, a pH change, a concentration change of a denaturant, or a salt concentration change, wherein,
the analysis method satisfies at least one of the following conditions (A) to (E):
(A) a primer set is used in the amplification step,
the primer set is a combination of a first primer for a polymerase that performs priming at the 3' end of a primer to synthesize the target sequence and a second primer for the polymerase to synthesize the complementary strand,
between the first primer and the second primer, a primer that initiates synthesis of a sequence to be hybridized with the probe is a turnback primer including a primer region and a probe region being directly or indirectly linked to the 5' end of the primer region, and the other primer is any primer having an ability of causing the polymerase to perform priming at the 3' end,
in the amplification step, the turnback primer extends the target sequence or the complementary sequence from the primer region, and
in the detection step, the turnback primer also serves as the probe, and association between the probe region and the target sequence or the complementary sequence, which occurred by turning the probe region of the turnback primer in a molecule of the amplification product back toward the extended target sequence or the extended complementary sequence is detected;
(B) the detection step comprises a step of detecting association between the probe and the amplification product with a temperature change while changing a temperature from a high temperature point to a low temperature point;
(C) in the amplification step, a primer set is used, the primer set is a combination of a first primer that performs priming of a polymerase that synthesizes the target sequence and a second primer that performs priming of a polymerase that synthesizes the complementary strand, and
between the first primer and the second primer, a primer that performs priming of a polymerase that synthesizes a sequence to be hybridized with the probe or the probe region in the condition (A) is used in an amount higher than the other primer;
(D) in the probe, the length of a region to be hybridized with the target sequence or the complementary sequence is 6 to 100 nucleotides; and
(E) the primer set is a combination of a first primer that performs priming of a polymerase that synthesizes the target sequence and a second primer that performs priming of a polymerase that synthesizes the complementary sequence,
the first primer has the same sequence as an upstream region at any site of the template nucleic acid,
the second primer has a complementary sequence to a downstream region at any site of the template nucleic acid, and
the analysis method further satisfies the condition (e1) or (e2):
(e1) the probe hybridizes with the target sequence, the 3' end of the upstream region in the template nucleic acid is positioned in the vicinity of the upstream side of the 5' end of a region to be hybridized with the probe in the template sequence, and the 5' end of the downstream region in the template nucleic acid is positioned in the vicinity of the downstream side of the 3' end of a region to be hybridized with the probe in the template sequence; and
(e2) the probe hybridizes with the complementary sequence, the 3' end of the upstream region in the template nucleic acid is positioned in the vicinity of the upstream side of the 5' end of a region that overlaps in sequence with the probe in the template nucleic acid, and the 5' end of the downstream region in the template nucleic acid is positioned in the vicinity of the downstream side of the 3' end of a region that overlaps in sequence with the probe in the template nucleic acid.

2. The analysis method according to claim 1, wherein
the analysis method is a method for analyzing a nucleic acid mutation present in a target nucleic acid mutation site in the template nucleic acid,
the target sequence has the nucleic acid mutation site in the template nucleic acid,
the amplification step is a step of amplifying the target sequence and a complementary sequence to the target sequence,
the detection step is a step of detecting association between the probe and the amplification product obtained in the amplification step with a temperature change, a pH change, a concentration change of a denaturant, or a salt concentration change or dissociation of an assembly between the probe and the amplification product with a temperature change, a pH change, a concentration change of a denaturant, or a salt concentration change,
the probe hybridizes with the target sequence or the complementary sequence, and
a nucleic acid mutation at the nucleic acid mutation site can be detected on the basis of a difference in dissociation temperature or association temperature between the probe that is mismatch to the nucleic acid mutation site and the probe that is fullmatch to the same or between the probe that is mismatch to the nucleic acid mutation site and the probe that is different mismatch having a different Tm.

3. The analysis method according to claim 2, satisfying the condition (B), wherein
the high temperature point and the low temperature point satisfy the following condition (b1) or (b2):
(b1) the high temperature point is defined as any temperature point in a higher temperature region than the Tm in hybridization between "the probe" and "the target sequence having a nucleic acid mutation site to which the probe shows mismatch", and the low temperature point is defined as any temperature point in a lower temperature region than the Tm in hybridization between "the probe" and "the target sequence having a nucleic acid mutation site to which the probe shows fullmatch"; and
(b2) in the case where the probe shows mismatch to the nucleic acid mutation site and the probe shows different mismatch having a different Tm, the high temperature point is any temperature point in a higher temperature region than a relatively low Tm between the two Tms, and the lower temperature point is any temperature point in a lower temperature region than the relatively high Tm between the two Tms.

4. The analysis method according to claim 2 or 3, satisfying the condition (E), wherein
the any site is the nucleic acid mutation site,
the first primer has the same sequence as the upstream region of the nucleic acid mutation site in the template nucleic acid, and
the second primer has a complementary sequence to the downstream region of the nucleic acid mutation site in the template nucleic acid.

5. The analysis method according to any one of claims 1 to 4, wherein
a method of the amplification in the amplification step is PCR.

6. The analysis method according to any one of claims 1 to 5, wherein
the probe is a fluorogenic probe.

7. The analysis method according to any one of claims 1 to 6, wherein
the probe includes at least two fluorescent atomic groups that exhibit an excitonic effect, and the two fluorescent atomic groups have a structure bound to the same base or the respective two bases adjacent to each other in the probe via linkers.

8. The analysis method according to any one of claims 1 to 5, wherein
the detention step is performed in the presence of an intercalator, and
the intercalator is a fluorescent dye that develops color by intercalation of the intercalator into a double helix structure formed by hybridization of the probe.

9. The analysis method according to any one of claims 1 to 8, satisfying any one of the following combinations of the conditions (A) to (E):
the combination of the conditions (A) and (B);
the combination of the conditions (A), (B), and (C);
the combination of the conditions (A), (B), (C), and (D);
the combination of the conditions (A), (B), (C), (D), and (E);
the combination of the conditions (A) and (C);
the combination of the conditions (A), (C), and (D);
the combination of the conditions (A), (C), (D), and (E);
the combination of the conditions (A) and (D);
the combination of the conditions (A), (D), and (E);
the combination of the conditions (A) and (E);
the combination of the conditions (B) and (C);
the combination of the conditions (B), (C), and (D);
the combination of the conditions (B), (C), (D), and (E);
the combination of the conditions (B) and (D);
the combination of the conditions (B), (D), and (E); and
the combination of the conditions (B) and (E).

10. The analysis method according to any one of claims 1 to 9, wherein
the detection step is performed in the presence of at least one additive selected from the group consisting of DMSO, betaine, urea, formamide, and formalin.

11. The analysis method according to any one of claims 1 to 10, satisfying the condition (A) and further comprising:
a step of performing the amplification step in the presence of a nickase or adding a nickase after the amplification step to design a nickase recognition sequence in "the turnback primer or a nucleic acid strand synthesized of the turnback primer" and nick a complementary strand to "the turnback primer or the nucleic acid strand synthesized of the turnback primer".

12. The analysis method according to any one of claims 1 to 11, satisfying the condition (A), wherein
in the turnback primer, the probe region is indirectly linked to the 5' end of the primer region via an intervening sequence.

13. The analysis method according to claim 12, wherein
the nickase recognition site according to claim 11 is present in the intervening sequence according to claim 12.

14. The analysis method according to any one of claims 1 to 13, satisfying the condition (A) and further satisfying the condition (a1), (a1), (a2), or (a2'):
(a1) in the case where the first primer is the turnback primer and an intervening sequence is absent between the primer region (Ac') and the probe region (B'), (X - Y)/(X) is in the range from -1 to 1, assuming that the number of bases of the primer region (Ac') is X, and the number of bases of a region sandwiched between a region (A) to be hybridized with the primer region (Ac') and a region (B) to be hybridized with the probe (B') hybridizes in the complementary sequence is Y;
(a1') in the case where the first primer is the turnback primer, and an intervening sequence is present between the primer region (Ac') and the probe region (B'), [X - (Y - Y')]/X is in the range from -1 to 1, assuming that the number of bases of the primer region (Ac') is X, the number of bases of a region sandwiched between a region (A) to be hybridized with the primer region (Ac') and a region (B) to be hybridized with the probe (B') hybridizes is Y, and the number of bases of the intervening sequence is Y';
(a2) in the case where the second primer is the turnback primer, and an intervening sequence is absent between the primer region (cAc') and the probe region (cB'), (X - Y) /(X) is in the range from -1 to 1, assuming that the number of bases of the primer region (cAc') is X, and the number of bases of a region sandwiched between a region (cA) to be hybridized with the primer region (cAc') and a region (cB) to be hybridized with the probe (cB') in the target sequence is Y; and
(a2') in the case where the second primer is the turnback primer, and an intervening sequence is present between the primer region (cAc') and the probe region (cB'), [X - (Y - Y')]/X is in the range from -1 to 1, assuming that the number of bases of the primer region (cAc') is X, the number of bases of a region sandwiched between a region (cA) to be hybridized with the primer region (cAc') and a region (cB) to be hybridized with the probe (cB') in the target sequence is Y, and the number of bases in the intervening sequence is Y'.

15. The analysis method according to any one of claims 1 to 14, satisfying the condition (C), wherein
X is in the range of 1 < X ≤ 10⁶, assuming that the concentration of a primer (M) is higher than that of the other primer (S), and a molar ratio (M : S) is X : 1.

16. The analysis method according to any one of claims 1 to 15, wherein
the probe is an FRET probe comprising at least two fluorescent atomic group pairs that exhibit an excitonic effect or an FRET probe comprising at least one fluorescent atomic group pair that exhibits an excitonic effect and a dye that exhibits no excitonic effect, and
the two fluorescent atomic groups of each of the fluorescent dy moiety pairs are bound to the same base or the respective bases that are adjacent to each other in the probe via linkers and are arranged to exhibit an FRET effect in the probe.

17. The analysis method according to claim 16, wherein
an emission peak wavelength of one (hereinafter referred to as the "fluorescent atomic group pair A") of the two fluorescent atomic group pairs is lower than the other (hereinafter referred to as the "fluorescent atomic group pair B"), and
the fluorescent atomic group pairs A and B exhibit an FRET effect.

18. The analysis method according to claim 16 or 17, wherein
a base having the fluorescent atomic group pair A and a base having the fluorescent atomic group pair B are contained in the probe at a distance at which the fluorescent atomic group pairs A and B exhibit an FRET effect.

19. The analysis method according to any one of claims 16 to 18, wherein
the distance between the base having the fluorescent atomic group pair A and the base having the fluorescent atomic group pair B is from 1 to 11 bases.

20. The analysis method according to any one of claims 16 to 19, wherein
the base having the fluorescent atomic group pair that exhibits an excitonic effect has a structure represented by the following formula (16), (16b), (17), or (17b):

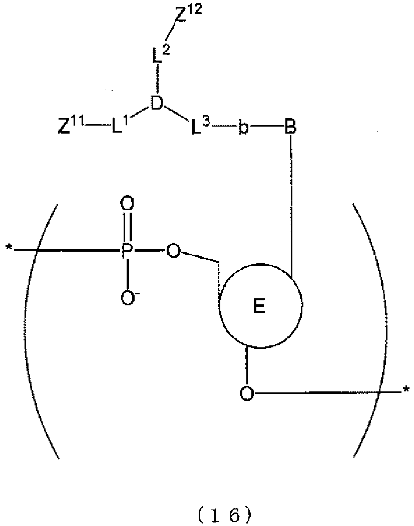

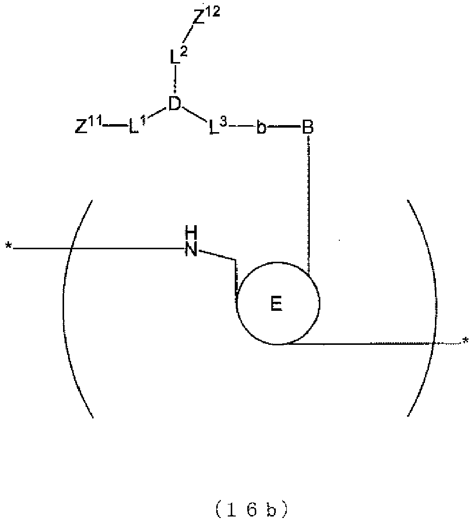

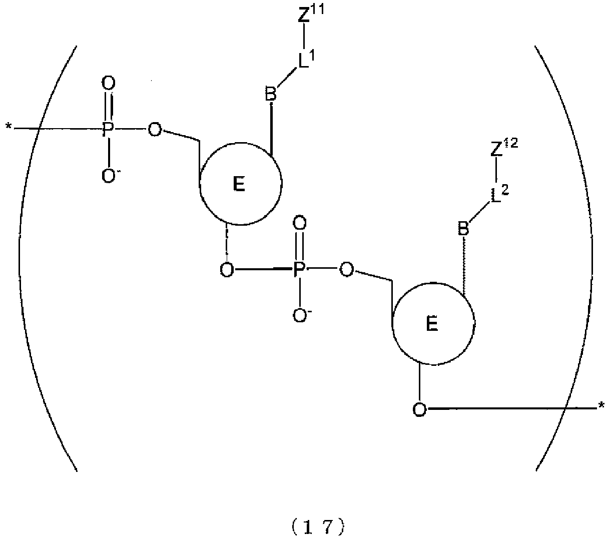

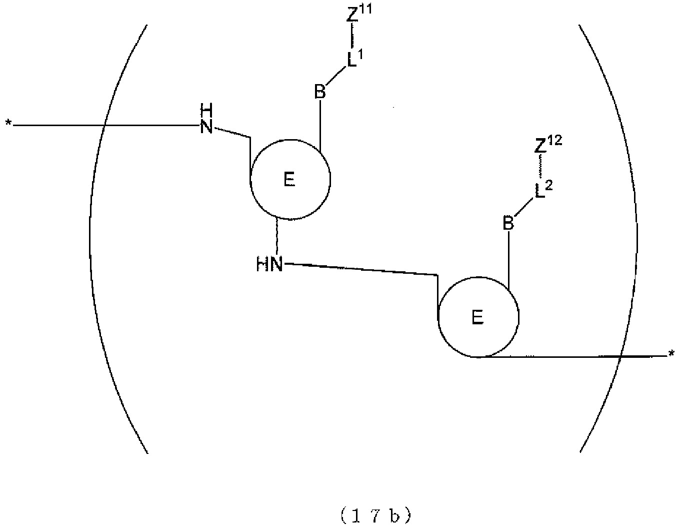

21. The analysis method according to any one of claims 1 to 20, wherein
the probe comprises at least one of a non-nucleotide residue, a modified nucleotide residue, and a non-natural main backbone.

22. The analysis method according to claim 21, wherein
the non-natural main backbone is at least one of LNA, PNA, and a nucleic acid having a modified phosphodiester bond.

23. The analysis method according to any one of claims 1, 2, and 4 to 22, wherein
the analysis method satisfies at least one of the conditions (A) and (C) to (E), and
the detection step is a step of detecting dissociation of an assembly between the probe and the amplification product with a temperature change while changing a temperature from a low temperature point to a high temperature point.

24. An oligonucleotide for performing the analysis method according to any one of claims 1 to 23.

25. A device for performing the analysis method according to any one of claims 1 to 24.
